# EUROPEAN PATENT SPECIFICATION

(11) **EP 3 249 404 B1**
(45) Date of publication and mention of the grant of the patent: **02.10.2019**
(21) Application number: 16740308.8
(22) Date of filing: 22.01.2016
(51) Int. Cl.: G01N 33/543, C12M 1/00, C12Q 1/68, G01N 35/02, B01L 3/00

(54) **TARGET ANALYSIS APPARATUS AND TARGET ANALYSIS METHOD**
ZIELANALYSEVORRICHTUNG UND ZIELANALYSEVERFAHREN
APPAREIL D'ANALYSE DE CIBLE ET PROCÉDÉ D'ANALYSE DE CIBLE

(30) Priority: 22.01.2015 JP 2015010514; 22.07.2015 JP 2015145280
(43) Date of publication of application: 29.11.2017
(73) Proprietor: NEC Solution Innovators, Ltd., Koto-ku, Tokyo 136-8627 (JP)
(72) Inventor: WAGA, Iwao, Tokyo 136-8627 (JP); HORII, Katsunori, Tokyo 136-8627 (JP); AKITOMI, Jou, Tokyo 136-8627 (JP); KANEKO, Naoto, Tokyo 136-8627 (JP); YOSHIDA, Yoshihito, Tokyo 136-8627 (JP)
(74) Representative: Betten & Resch
(86) International application number: PCT/JP2016/051926
(87) International publication number: WO 2016/117701

(56) References cited:
- EP-A1- 0 141 547
- EP-A1- 0 311 604
- WO-A1-84/02004
- WO-A1-92/18844
- WO-A1-2009/076347
- JP-A- H03 170 060
- JP-A- H03 170 060
- JP-A- H11 514 849
- JP-A- H11 514 849
- JP-A- 2002 541 478
- JP-A- 2002 541 478
- JP-A- 2007 518 994
- JP-A- 2007 518 994
- JP-A- 2014 006 226
- JP-A- 2014 006 226

## Description

### Technical Field

The present invention relates to a target analysis tool and a target analysis method.

### Background Art

In general, target analysis is performed using binding substances having binding affinity to a target, and the presence or absence of the target or the amount of the target can be analyzed by causing the binding substances to form conjugates with the target and detecting the thus-formed conjugates directly or indirectly (Patent Documents 1 and 2). JP H03 170060 describes an apparatus for testing biological molecular indicators in blood using a tubular device with compartments.

However, an analysis method performed using binding substances in the above-described manner requires a process of separating the binding substances bound to the target and the binding substances not bound to the target. Accordingly, it is difficult to perform a series of processes and detect the target in a single analysis tool.

### Citation List

### Patent Document(s)

Patent Document 1: Japanese Translation of PCT International Application Publication No. JP-T-2014-507670
Patent Document 2: Japanese Translation of PCT International Application Publication No. JP-T-2007-518994

### Brief Summary of the Invention

### Problem to be Solved by the Invention

With the foregoing in mind, it is an object of the present invention to provide a target analysis tool for easy analysis of a target.

### Means for Solving Problem

The present disclosure provides a first target analysis tool including: a first chamber; a second chamber; and a third chamber. The first chamber, the second chamber, and the third chamber are disposed continuously in this order. The first chamber contains, as a first reagent, an immobilized first binding substance obtained by immobilizing, on a carrier, a first binding substance that binds to a target. The second chamber contains, as a second reagent, a labeled second binding substance obtained by binding a labeling substance to a second binding substance that binds to the first binding substance. The third chamber is a detection section in which the labeled second binding substance is detected. A first partition wall is provided between the first chamber and the second chamber. A second partition wall is provided between the second chamber and the third chamber. The first chamber is configured so that a specimen holder having a specimen can be inserted from an outside of the first chamber to an inside of the first chamber. The first partition wall is a partition wall that is broken upon contact with a tip of the specimen holder inserted into the first chamber. The second partition wall is a porous partition wall through which the immobilized first binding substance cannot pass and the labeled second binding substance can pass.

The present disclosure also provides a first target analysis method using the first target analysis tool. The first target analysis method includes the steps of: inserting a specimen holder holding a specimen into the first chamber of the target analysis tool; binding a target in the specimen to the immobilized first binding substance as the first reagent by causing the specimen to be in contact with the first reagent in the first chamber; introducing a mixture of the specimen and the first reagent in the first chamber to the second chamber by bringing the specimen holder in the first chamber into contact with the first partition wall between the first chamber and the second chamber to break the partition wall; binding the immobilized first binding substance to the labeled second binding substance as the second reagent by causing the mixture of the specimen and the first reagent to be in contact with the second reagent in the second chamber; introducing an unbound labeled second binding substance to the third chamber through the second partition wall between the second chamber and the third chamber; and detecting the labeling substance in the labeled second binding substance in the third chamber.

The present disclosure also provides a second target analysis tool including: a first chamber; a second chamber; and a third chamber. The first chamber, the second chamber, and the third chamber are disposed continuously in this order. The first chamber contains, as a first reagent, a labeled first binding substance obtained by binding a labeling substance to a first binding substance that binds to a target. The second chamber contains, as a second reagent, an immobilized second binding substance obtained by immobilizing, on a carrier, a second binding substance that binds to the first binding substance. The third chamber is a detection section in which the labeled first binding substance is detected. A first partition wall is provided between the first chamber and the second chamber. A second partition wall is provided between the second chamber and the third chamber. The first chamber is configured so that a specimen holder having a specimen can be inserted from an outside of the first chamber to an inside of the first chamber. The first partition wall is a partition wall that is broken upon contact with a tip of the specimen holder inserted into the first chamber or a porous partition wall through which contents in the first chamber can pass to enter the second chamber. The second partition wall is a porous partition wall through which the immobilized second binding substance cannot pass and the labeled first binding substance can pass.

The present disclosure also provides a second target analysis method using the second target analysis tool. The second target analysis method includes the steps of: inserting a specimen holder holding a specimen into the first chamber of the target analysis tool; binding a target in the specimen to the labeled first binding substance as the first reagent to form a first conjugate by causing the specimen to be in contact with the first reagent in the first chamber; binding an unbound labeled first binding substance not bound to the target in a mixture of the specimen and the first reagent to the immobilized second binding substance as the second reagent by causing the mixture into contact with the second reagent in the second chamber; introducing the first conjugate to the third chamber through the second partition wall between the second chamber and the third chamber; and detecting the labeled first binding substance in the first conjugate in the third chamber.

The present disclosure also provides a third target analysis tool including: a first chamber; a second chamber; and a third chamber. The first chamber, the second chamber, and the third chamber are disposed continuously in this order. The first chamber contains, as a first reagent, a labeled second binding substance obtained by binding a labeling substance to a second binding substance that binds to a first binding substance that binds to a target. The second chamber contains, as a second reagent, an immobilized first binding substance obtained by immobilizing the first binding substance on a carrier. The third chamber is a detection section in which the labeled second binding substance is detected. A first partition wall is provided between the first chamber and the second chamber. A second partition wall is provided between the second chamber and the third chamber. The first chamber is configured so that a specimen holder having a specimen can be inserted from an outside of the first chamber to an inside of the first chamber. The first partition wall is a partition wall that is broken upon contact with a tip of the specimen holder inserted into the first chamber or a porous partition wall through which contents in the first chamber can pass to enter the second chamber. The second partition wall is a porous partition wall through which the immobilized first binding substance cannot pass and the labeled second binding substance can pass.

The present disclosure also provides a third target analysis method using the third target analysis tool. The third analysis method includes the steps of: inserting a specimen holder holding a specimen into the first chamber of the target analysis tool; binding a target in the specimen to the immobilized first binding substance as the second reagent and also binding an unbound immobilized first binding substance not bound to the target to the labeled second binding substance as the first reagent by causing a mixture of the specimen and the first reagent to be in contact with the second reagent in the second chamber; introducing an unbound labeled second binding substance to the third chamber through the second partition wall between the second chamber and the third chamber; and detecting the labeling substance in the labeled second binding substance in the third chamber.

The present invention also provides a fourth target analysis tool including: a first chamber; a second chamber; and a third chamber. The first chamber, the second chamber, and the third chamber are disposed continuously in this order. The first chamber contains, as a first reagent, an immobilized second binding substance obtained by immobilizing, on a carrier, a second binding substance that binds to a first binding substance that binds to a target. The second chamber contains, as a second reagent, a labeled first binding substance obtained by binding a labeling substance to the first binding substance. The third chamber is a detection section in which the labeled first binding substance is detected. A first partition wall is provided between the first chamber and the second chamber. A second partition wall is provided between the second chamber and the third chamber. The first chamber is configured so that a specimen holder having a specimen can be inserted from an outside of the first chamber to an inside of the first chamber. The first partition wall is a partition wall that is broken upon contact with a tip of the specimen holder inserted into the first chamber. The second partition wall is a porous partition wall through which the immobilized second binding substance cannot pass and the labeled first binding substance not bound to the immobilized second binding substance can pass, wherein the first binding substance in the labeled first binding substance is an aptamer, and the second binding substance in the immobilized second binding substance is a nucleic acid molecule complementary to the aptamer.

The present invention also provides a fourth target analysis method using the fourth target analysis tool. The fourth target analysis method includes the steps of introducing a specimen and the first reagent to the second chamber by inserting a specimen holder holding the specimen into the first chamber of the target analysis tool and then bringing the specimen holder into contact with the first partition wall between the first chamber and the second chamber; binding a target in the specimen to the labeled first binding substance as the second reagent to form a first conjugate and also binding an unbound labeled first binding substance not bound to the target to the immobilized second binding substance as the first reagent by causing the specimen and the first reagent to be in contact with the second reagent in the second chamber; introducing the first conjugate to the third chamber through the second partition wall between the second chamber and the third chamber; and detecting the labeled first binding substance in the first conjugate in the third chamber.

### Effects of the Invention

According to the target analysis tool of the present invention, a target in a specimen can be analyzed easily.

### Brief Description of Drawings

FIG. 1 is a schematic view showing an example of the first target analysis tool.
FIGs. 2A to 2C schematically show an example of an analysis method using the first target analysis tool.
FIG. 3 is a schematic view showing an example of the second target analysis tool.
FIGs. 4A to 4C schematically show an example of an analysis method using the second target analysis tool.
FIG. 5 is a schematic view showing another example of the second target analysis tool.
FIG. 6 is a graph showing the absorbance measured in Example 1.
FIG. 7 is a graph showing the absorbance measured in Example 1.
FIG. 8 is a graph showing the proportion of collected labeled complementary strands in Example 2.
FIG. 9 is a schematic view showing still another example of the second target analysis tool.
FIG. 10 is a schematic view showing an example of the third target analysis tool.
FIGs. 11A to 11C schematically show an example of an analysis method using the third target analysis tool.
FIG. 12 is a schematic view showing an example of the fourth target analysis tool of the present invention.
FIGs. 13A to 13C schematically show an example of an analysis method using the fourth target analysis tool of the present invention.
FIG. 14 is a graph showing the amount of light emission in Example 3.
FIG. 15 is a graph showing the amount of light emission in Example 4.
FIG. 16 is a graph showing the amount of light emission in Example 5 of the present invention.
FIG. 17 is a graph showing the amount of light emission in Example 6.

### Mode for Carrying out the Invention

In the first and third target analysis tools, the carrier in the immobilized first binding substance may be a bead, for example.

In the first and third target analysis tools, the labeling substance in the labeled second binding substance may be at least one substance selected from the group consisting of enzymes, nucleic acids, fluorescent substances, dye substances, luminescent substances, radioactive substances, and electron donors, for example.

In the first and third target analysis tools, the specimen holder may include a rod-shaped grip section and a holding section for holding a specimen, and the holding section may be provided at a tip of the grip section, for example.

In the first and third target analysis tools, the first binding substance in the immobilized first binding substance may be an aptamer, for example.

In the first and third target analysis tools, the second binding substance in the labeled second binding substance may be a nucleic acid molecule complementary to the aptamer, for example.

In the first and third target analysis tools, the labeling substance in the labeled second binding substance may be a nucleic acid molecule exhibiting a catalytic function, for example.

In the first and third target analysis, the nucleic acid molecule exhibiting a catalytic function may be at least one of a DNAzyme and an RNAzyme, for example.

In the first and third target analysis tools the second chamber may contain an adsorbent carrier that adsorbs at least one of proteins and lipids, for example.

In the first and third target analysis tools, the adsorbent carrier may be a silica bead, for example.

In the first and third target analysis tools, the first binding substance in the immobilized first binding substance may be an antibody against the target as an antigen, and the second binding substance in the labeled second binding substance may be an antibody against the first binding substance as an antigen, for example.

The first and third target analysis tools may be configured so that, for example, it includes an outer cylinder and an inner cylinder, the inner cylinder can be housed inside the outer cylinder, the inner cylinder has the first chamber and the second chamber, and when the inner cylinder is housed inside the outer cylinder, there is a space between a bottom of the outer cylinder and a bottom of the inner cylinder.

The first and third target analysis tools may be configured so that, for example, it further includes a pretreatment chamber disposed continuously with the first chamber on a side opposite to the second chamber, wherein the pretreatment chamber is configured so that the specimen holder can be inserted from an outside of the pretreatment chamber to an inside of the pretreatment chamber, the pretreatment chamber contains an extractant for extracting a component from the specimen, a partition wall is provided between the pretreatment chamber and the first chamber, and the partition wall is a partition wall that is broken upon contact with the tip of the specimen holder inserted into the pretreatment chamber.

In the first and third target analysis tools, the pretreatment chamber may include a cover for an opening of the pretreatment chamber on a side opposite to the partition wall between the pretreatment chamber and the first chamber, and the cover may be configured so that the specimen holder can be inserted from an outside of the cover to an inside of the pretreatment chamber, for example.

In the second analysis tool, the first binding substance in the labeled first binding substance may be an aptamer, and the second binding substance in the immobilized second binding substance may be a nucleic acid molecule complementary to the aptamer, for example. In the fourth target analysis tools of the present invention, the first binding substance in the labeled first binding substance is an aptamer, and the second binding substance in the immobilized second binding substance is a nucleic acid molecule complementary to the aptamer.

In the second analysis tool as described herein and fourth target analysis tools of the present invention, the labeling substance in the labeled first binding substance may be at least one substance selected from the group consisting of enzymes, nucleic acids, fluorescent substances, dye substances, luminescent substances, radioactive substances, and electron donors, for example.

In the second analysis tool as described herein and fourth target analysis tools of the present invention, the labeling substance in the labeled first binding substance may be a nucleic acid molecule exhibiting a catalytic function, for example.

In the second analysis tool as described herein and fourth target analysis tools of the present invention, the nucleic acid molecule exhibiting a catalytic function may be at least one of a DNAzyme and an RNAzyme, for example.

In the second analysis tool as described herein and fourth target analysis tools of the present invention, the carrier in the immobilized second binding substance may be a bead, for example.

In the second analysis tool as described herein and fourth target analysis tools of the present invention, the carrier in the immobilized second binding substance may be an inner wall of the second chamber, for example.

In the second analysis tool as described herein and fourth target analysis tools of the present invention, the specimen holder may include a rod-shaped grip section and a holding section for holding a specimen, and the holding section may be provided at a tip of the grip section, for example.

In the second analysis tool as described herein and fourth target analysis tools of the present invention, the second chamber may contain an adsorbent carrier that adsorbs at least one of proteins and lipids, for example.

In the second analysis tool as described herein and fourth target analysis tools of the present invention, the adsorbent carrier may be a silica bead, for example.

The second analysis tool as described herein and fourth target analysis tools of the present invention may be configured so that, for example, it includes an outer cylinder and an inner cylinder, the inner cylinder can be housed inside the outer cylinder, the inner cylinder has the first chamber and the second chamber, and when the inner cylinder is housed inside the outer cylinder, there is a space between a bottom of the outer cylinder and a bottom of the inner cylinder.

The second analysis tool as described herein and fourth target analysis tools of the present invention may be configured so that, for example, it further includes a pretreatment chamber disposed continuously with the first chamber on a side opposite to the second chamber, wherein the pretreatment chamber is configured so that the specimen holder can be inserted from an outside of the pretreatment chamber to an inside of the pretreatment chamber, the pretreatment chamber contains an extractant for extracting a component from the specimen, a partition wall is provided between the pretreatment chamber and the first chamber, and the partition wall is a partition wall that is broken upon contact with the tip of the specimen holder inserted into the pretreatment chamber.

In the second analysis tool as described herein and fourth target analysis tools of the present invention, the pretreatment chamber may include a cover for an opening of the pretreatment chamber on a side opposite to the partition wall between the pretreatment chamber and the first chamber, and the cover may be configured so that the specimen holder can be inserted from an outside of the cover to an inside of the pretreatment chamber, for example.

The second and third target analysis methods may further include the step of introducing the mixture of the specimen and the first reagent in the first chamber to the second chamber by bringing the specimen holder in the first chamber into contact with the first partition wall between the first chamber and the second chamber to break the partition wall, for example.

The third target analysis method may further include the step of inserting the specimen holder into the first chamber and then mixing the specimen and the first reagent, for example.

The fourth target analysis method of the present invention may further include the steps of: mixing the specimen and the first reagent by inserting the specimen holder into the first chamber; and introducing a mixture of the specimen and the first reagent in the first chamber to the second chamber by bringing the specimen holder in the first chamber into contact with the first partition wall between the first chamber and the second chamber to break the partition wall, for example.

### [First Target Analysis Tool and First Target Analysis Method]

The first target analysis tool and the first target analysis method will be described specifically below. Unless otherwise stated, descriptions regarding the second to fourth target analysis tools and the second to fourth target analysis methods to be described below also apply to the first target analysis tool and the first target analysis method.

The first target analysis tool is, as described above, a target analysis tool including: a first chamber; a second chamber; and a third chamber, wherein the first chamber, the second chamber, and the third chamber are disposed continuously in this order, the first chamber contains, as a first reagent, an immobilized first binding substance obtained by immobilizing, on a carrier, a first binding substance that binds to a target, the second chamber contains, as a second reagent, a labeled second binding substance obtained by binding a labeling substance to a second binding substance that binds to the first binding substance, the third chamber is a detection section in which the labeled second binding substance is detected, a first partition wall is provided between the first chamber and the second chamber, a second partition wall is provided between the second chamber and the third chamber, the first chamber is configured so that a specimen holder having a specimen can be inserted from an outside of the first chamber to an inside of the first chamber, the first partition wall is a partition wall that is broken upon contact with a tip of the specimen holder inserted into the first chamber, and the second partition wall is a porous partition wall through which the immobilized first binding substance cannot pass and the labeled second binding substance can pass.

The first target analysis method is, as described above, a target analysis method using the first target analysis tool, including the steps of inserting a specimen holder holding a specimen into the first chamber of the target analysis tool; binding a target in the specimen to the immobilized first binding substance as the first reagent by causing the specimen to be in contact with the first reagent in the first chamber; introducing a mixture of the specimen and the first reagent in the first chamber to the second chamber by bringing the specimen holder in the first chamber into contact with the first partition wall between the first chamber and the second chamber to break the partition wall; binding the immobilized first binding substance to the labeled second binding substance as the second reagent by causing the mixture of the specimen and the first reagent to be in contact with the second reagent in the second chamber; introducing an unbound labeled second binding substance to the third chamber through the second partition wall between the second chamber and the third chamber; and detecting the labeling substance in the labeled second binding substance in the third chamber.

As described herein, first, in the first chamber, a target in a specimen binds to the immobilized first binding substances as the first reagent. Then, the specimen holder breaks the first partition wall between the first chamber and the second chamber, thereby allowing the mixture of the specimen and the first reagent in the first chamber to be introduced to the second chamber. In the second chamber, the labeled second binding substances bind to, among the immobilized first binding substances, those not bound to the target. The second partition wall between the second chamber and the third chamber is a porous partition wall through which the immobilized first binding substances cannot pass and the labeled second binding substances can pass. Accordingly, the immobilized first binding substances do not pass through the partition wall and thus remain in the second chamber. That is, the labeled second binding substances bound to the immobilized first binding substances remain in the second chamber without moving to the third chamber. On the other hand, the labeled second binding substances that are in a free state without being bound to the immobilized first binding substances are introduced to the third chamber through the partition wall. The target analysis tool of the present invention can contain a known amount of the labeled second binding substances, for example. Accordingly, the amount of the labeled second binding substances not bound to the immobilized first binding substances indirectly corresponds to the amount of the target in the specimen. Thus, by detecting the unbound labeled second binding substances introduced to the third chamber, the presence or absence of the target or the amount of the target in the specimen can be analyzed indirectly.

The term "analysis" as used in the present invention may mean, for example, qualitative analysis to determine the presence or absence of a target or quantitative analysis to determine the amount of a target.

The first binding substances that bind to a target to be used described herein are not limited to a particular type of binding substances, as long as they bind to the target, for example. Specifically, the first binding substances according to the invention are aptamers. The first target analysis tool and the first target analysis method will be described below with reference to an example where the first target analysis tool is in a first A-form that uses aptamers.

In the first A-form, the first binding substances in the immobilized first binding substances are aptamers. The aptamers are not limited as long as they can bind to the target. The aptamer may be, for example, a DNA aptamers, an RNA aptamers, or a chimera aptamer containing DNA and RNA. The aptamer may consist of a natural nucleic acid or a non-natural nucleic acid, or may contain a natural nucleic acid and a non-natural nucleic acid. The aptamer may be a modified aptamer, for example. The aptamer may be single-stranded, for example.

The second binding substances in the labeled second binding substances are not limited as long as they can bind to the aptamers, and may be, for example, nucleic acid molecules complementary to the aptamers (also referred to as "complementary nucleic acid molecules" hereinafter). The nucleic acid molecules complementary to the aptamers are not limited to those that are 100% complementary to the aptamers, and may have complementarity sufficient to hybridize to the aptamers or a partial sequence thereof, for example. The complementarity may be, for example, at least 95%, at least 96%, at least 97%, at least 98%, at least 99%, or 100%. By using the complementary nucleic acid molecules as the second binding substances, a reaction between the first conjugates and the labeled second binding substances can be caused easily, for example. Thus, the reaction time can be shortened, and also, a higher sensitivity and a broader dynamic range can be achieved. It is preferable that the complementary nucleic acid molecules do not bind to the target, for example.

The carriers in the immobilized first binding substances may be beads, for example. The material of the beads is not particularly limited, and may be a polymer such as agarose, sepharose, or cellulose, for example. The beads may be magnetic beads, for example. The magnetic beads may be, for example, beads consisting of a magnetic material, beads containing the magnetic material, or beads coated with the magnetic material. The magnetic material may be, for example, a magnetizable substance, and specific examples thereof include γFe₂O₃ and Fe₃O₄. The shape of the beads is not particularly limited, and may be spherical such as completely spherical, for example. The average diameter of the beads is not particularly limited, and may be from 1 to 10 µm, 10 to 100 µm, or 100 to 1000 µm, for example. The carriers may be formed using a resin such as Sepharose or Sephadex, for example.

In the immobilized first binding substances, the amount of the aptamers to be immobilized on the carriers is not particularly limited, and may be, for example, from 0.1 fmol to 100 pmol, 1 fmol to 10 pmol, or 10 fmol to 1 pmol per mm² surface area of the carrier.

The labeling substances in the labeled second binding substances may be catalytic nucleic acid molecules exhibiting a catalytic function or an enzyme, for example. The enzyme may be, for example, luciferase, peroxidase such as horseradish peroxidase (HRP), or alkaline phosphatase. In the first A-form, the labeling substances preferably are, for example, catalytic nucleic acid molecules as described above, similarly to the first binding substances and the second binding substances.

The catalytic nucleic acid molecules are not particularly limited, and may be, for example, a DNAzyme or an RNAzyme. The catalytic nucleic acid molecules in the labeled second binding substances preferably exhibit a catalytic function regardless of whether the target has been bound to the labeled second binding substances, for example. The form of the binding between the binding substance and the catalytic nucleic acid molecule is not particularly limited, and may be phosphodiester bond, for example. The binding substance and the catalytic nucleic acid molecule may be bound together directly or indirectly via a linker, for example. The linker may be a nucleic acid molecule consisting of at least one of DNA and RNA, for example. The catalytic nucleic acid molecules preferably are single-stranded, for example. When the labeling substance is the catalytic nucleic acid molecule, the catalytic nucleic acid molecule may serve both as the labeling substance and the second binding substance. That is, if the catalytic nucleic acid molecule is complementary to the first binding substance, the catalytic nucleic acid molecule can be used as the labeled second binding substance.

The specimen is not particularly limited, and may be a food-derived specimen, for example. Examples of the food-derived specimen include foods, food ingredients, food additives, attached substances in food-processing factories, kitchens, etc., and liquid obtained after washing food-processing factories, kitchens, etc. The form of the specimen is not particularly limited, and the specimen may be liquid or solid, for example. When the specimen is solid, the specimen may be in the form of a mixed solution, an extract, a dissolved solution, or the like prepared using a solvent, for example. The solvent is not particularly limited, and may be, for example, water, physiological saline, or a buffer. The specimen may or may not contain the target, or whether the specimen contains the target may be unknown, for example.

In the first A-form, aptamers can be used as the first binding substances, complementary nucleic acid molecules can be used as the second binding substances, and catalytic nucleic acid molecules can be used as the labeling substances, for example. Thus, the target analysis tool in the first A-form is thermostable and can be stored more easily, for example. It is also possible to use an enzyme such as luciferase, alkaline phosphatase, or peroxidase as the labeling substances, for example. Thus, it is possible to analyze a target with high sensitivity, for example.

The first partition wall between the first chamber and the second chamber is, as described above, a partition wall that is broken upon contact with the tip of the specimen holder. The first partition wall is, for example, the bottom of the first chamber, and it also can be said that the first partition wall is an upper part of the second chamber. The material, the properties, and the like of the first partition wall are not particularly limited as long as the first partition wall can be broken upon contact with the tip of the specimen holder. The first partition wall can be formed of a thin metal film (such as aluminum foil), paper, or synthetic fibers, for example.

The second partition wall between the second chamber and the third chamber is, as described above, a porous partition wall through which the immobilized first binding substances cannot pass and the labeled second binding substances can pass. The pore size of the second partition wall can be set as appropriate depending on the sizes of the immobilized first binding substances and the labeled second binding substances, for example. The pore size of the second partition wall is, for example, from 0.2 to 100 µm, 0.2 to 50 µm, or 0.5 to 10 µm.

When catalytic nucleic acid molecules are used as the labeling substances, it is preferable that the second chamber further contains, as the second reagent, adsorbent carriers that adsorb at least one of proteins and lipids. When the second chamber contains the adsorbent carriers, the adsorbent carriers adsorb proteins and lipids in the second chamber. This prevents, for example, proteins, lipids, etc. that may affect the catalytic function of the catalytic nucleic acid molecules from being introduced to the third chamber, so that the unbound labeled second binding substances in the third chamber can be detected more accurately. In the first A-form, the aptamers as the first binding substances that bind to a target and the complementary nucleic acid molecules as the second binding substances that bind to the first binding substances are both nucleic acids. Thus, by holding the proteins, lipids, etc., which are components other than nucleic acids, in the second chamber using the adsorbent carriers, target analysis can be carried out more accurately.

The material of the adsorbent carriers is not particularly limited, and may be, for example, silica, a crosslinked polymer having a porous structure, and active carbon. The shape of the adsorbent carriers is not particularly limited, and the adsorbent carriers may be beads, for example. Preferably, the adsorbent carriers are silica beads, for example. The size of the adsorbent carriers is not particularly limited. Preferably, the size of the adsorbent carriers is such that the adsorbent carriers cannot pass through the second partition wall (porous partition wall), for example. The adsorbent carriers may have the same size as the carriers in the immobilized first binding substances, for example.

When the labeling substances in the labeled second binding substances are the catalytic nucleic acid molecules or the enzyme, it is preferable that the third chamber further contains a substrate for the catalytic function of the catalytic nucleic acid molecules or the enzyme, for example. As the substrate, ATP and luciferin may be used in combination, or a luminol reaction solution may be used, for example.

An example of a target analysis method using a target analysis tool in the first A-form will be described specifically with reference to drawings.

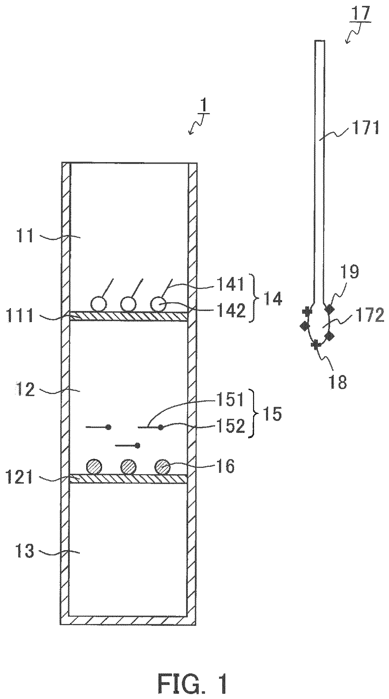

FIG. 1 schematically shows a target analysis tool in the first A-form and a target analysis method using this target analysis tool. A target analysis tool 1 includes a first chamber 11, a second chamber 12, and a third chamber 13. In the first chamber 11, immobilized aptamers 14 obtained by immobilizing aptamers 141 on beads 142 are disposed. In the second chamber 12, labeled complementary strands 15, which are obtained by adding a DNAzyme 152 to complementary strands 151 complementary to the aptamers 141, and silica beads 16 are disposed. A first partition wall 111 is provided between the first chamber 11 and the second chamber 12. A porous second partition wall 121 is provided between the second chamber 12 and the third chamber 13. A specimen holder 17 includes a rod-shaped grip section 171 and a specimen holding section 172, and the holding section 172 is provided at the tip of the grip section 171. The specimen holder 17 collects a specimen with the holding section 172. The target 18 in a specimen and contaminants 19 other than the target adhere to the holding section 172, for example.

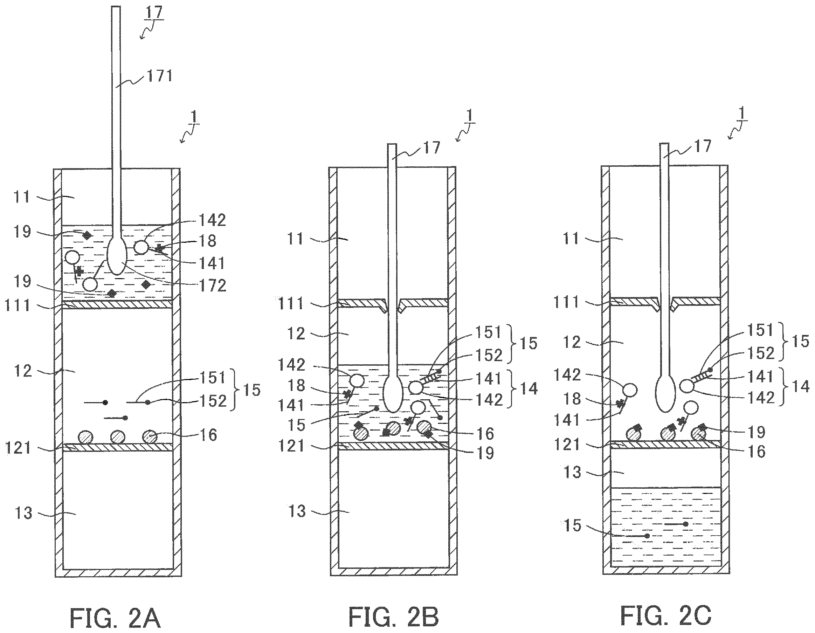

Next, an analysis method using the target analysis tool 1 shown in FIG. 1 will be described with reference to FIGs. 2A to 2C. FIGs. 2A to 2C schematically show the method of using the target analysis tool 1.

First, the specimen holder 17 with the specimen being held in the holding section 172 is inserted to the first chamber 11 of the target analysis tool 1, thereby binding the target 18 in the specimen to the aptamers 141 in the immobilized aptamers 14 in the first chamber 11. Conditions of the treatment for binding them are not particularly limited, and may be as follows, for example: the treatment temperature is from 4°C to 45°C and the treatment time is from 10 seconds to 10 minutes. The treatment for binding them preferably is performed in a liquid solvent, for example, and the liquid solvent may be, for example, an aqueous solvent such as water, a buffer solution, physiological saline, or a mixture thereof.

Next, the specimen holder 17 breaks the first partition wall 111, whereby the contents in the first chamber 11 are introduced to the second chamber 12. Then, the labeled complementary strands 15 are caused to bind to, among the aptamers 141 immobilized on the beads 142, those not bound to the target 18. Conditions of the treatment for binding them are not particularly limited, and may be as follows, for example: the treatment temperature is from 4°C to 45°C and the treatment time is from 10 seconds to 30 minutes.

The second partition wall 121 is a porous partition wall through which the immobilized aptamers 14 do not pass and the labeled complementary strands 15 pass. Accordingly, among the labeled complementary strands 15, only those not bound to the immobilized aptamers 14 pass through the second partition wall 121 to be introduced to the third chamber 13. In the second chamber 12, the silica beads 16 are disposed. Accordingly, for example, contaminants 19 such as proteins and lipids derived from the specimen are adsorbed onto the silica beads 16, whereby the contaminants 19 are prevented from being introduced to the third chamber 13. Then, in the third chamber 13, by subjecting the unbound labeled complementary strands 15 to measurement of the catalytic function of the DNAzyme 152, the target in the specimen can be analyzed indirectly. The method for measuring the catalytic function of the DNAzyme 152 can be determined as appropriate depending on the type of the DNAzyme 152.

The first target analysis tool may further include a pretreatment chamber, for example. As to the pretreatment chamber, reference can be made to the explanation to be given below, for example.

The first target analysis tool may include an outer cylinder and an inner cylinder, for example. In this case, the inner cylinder can be housed inside the outer cylinder, the inner cylinder has the first chamber and the second chamber, and when the inner cylinder is housed inside the outer cylinder, there is a space between the bottom of the outer cylinder and the bottom of the inner cylinder. When the first target analysis tool includes the outer cylinder and the inner cylinder, the outer cylinder can be used as the third chamber by detaching the inner cylinder after unbound labeled complementary strands have been introduced to the outer cylinder from the second chamber in the inner cylinder.

### [Second Target Analysis Tool and Second Target Analysis Method]

The second target analysis tool and the second target analysis method will be described specifically below. Unless otherwise stated, descriptions regarding the first, third, and fourth target analysis tools and the first, third, and fourth target analysis methods also apply to the second target analysis tool and the second target analysis method.

The second target analysis method is, as described above, a target analysis tool including: a first chamber; a second chamber; and a third chamber, wherein the first chamber, the second chamber, and the third chamber are disposed continuously in this order, the first chamber contains, as a first reagent, a labeled first binding substance obtained by binding a labeling substance to a first binding substance that binds to a target, the second chamber contains, as a second reagent, an immobilized second binding substance obtained by immobilizing, on a carrier, a second binding substance that binds to the first binding substance, the third chamber is a detection section in which the labeled first binding substance is detected, a first partition wall is provided between the first chamber and the second chamber, a second partition wall is provided between the second chamber and the third chamber, the first chamber is configured so that a specimen holder having a specimen can be inserted from an outside of the first chamber to an inside of the first chamber, the first partition wall is a partition wall that is broken upon contact with a tip of the specimen holder inserted into the first chamber or a porous partition wall through which contents in the first chamber can pass to enter the second chamber, and the second partition wall is a porous partition wall through which the immobilized second binding substance cannot pass and the labeled first binding substance (first conjugate) can pass.

The second target analysis method is, as described above, a target analysis method using the second target analysis tool, including the steps of: inserting a specimen holder holding a specimen into the first chamber of the target analysis tool; binding a target in the specimen to the labeled first binding substance as the first reagent to form a first conjugate by causing the specimen to be in contact with the first reagent in the first chamber; binding an unbound labeled first binding substance not bound to the target in a mixture of the specimen and the first reagent to the immobilized second binding substance as the second reagent by causing the mixture into contact with the second reagent in the second chamber; introducing the first conjugate to the third chamber through the second partition wall between the second chamber and the third chamber; and detecting the labeled first binding substance in the first conjugate in the third chamber.

As described herein, first, in the first chamber, a target in a specimen binds to the labeled first binding substances as the first reagent. Then, the mixture of the specimen and the first reagent in the first chamber is introduced to the second chamber. In the second chamber, the immobilized second binding substances bind to, among the labeled first binding substances, those not bound to the target. The second partition wall between the second chamber and the third chamber is a porous partition wall through which the immobilized second binding substances cannot pass and the labeled first binding substances can pass. Accordingly, the immobilized second binding substances do not pass through the partition wall and thus remain in the second chamber. That is, the labeled first binding substances bound to the immobilized second binding substances remain in the second chamber without moving to the third chamber. On the other hand, the labeled first binding substances that are in a free state without being bound to the immobilized second binding substance, i.e., the labeled first binding substances forming the first conjugates, are introduced to the third chamber through the partition wall. The target analysis tool can contain a known amount of the labeled first binding substances, for example. Accordingly, the amount of the labeled first binding substances not bound to the immobilized second binding substances indirectly corresponds to the amount of the target in the specimen. Thus, by detecting the unbound labeled first binding substances introduced to the third chamber, the presence or absence of the target or the amount of the target in the specimen can be analyzed indirectly.

The first partition wall may be a partition wall that is broken upon contact with the tip of the specimen holder inserted into the first chamber or a porous partition wall through which contents in the first chamber can pass to enter the second chamber, for example. In the former case, the contents in the first chamber can move to the second chamber by breaking the first partition wall, for example. In the latter case, the contents in the first chamber can move to the second chamber thorough the porous partition wall. In the former case, it is possible to use a member that can be broken in the above-described manner. In the latter case, it is possible to use a membrane having pores through which the contents in the first chamber can pass, for example.

The term "analysis" as used may mean, for example, qualitative analysis to determine the presence or absence of a target or quantitative analysis to determine the amount of a target.

As described above, the first binding substances that bind to a target to be used are not limited to a particular type of binding substances, as long as they bind to the target, for example. Specifically, the first binding substances may be aptamers or antibodies, for example. The second target analysis tool and the second target analysis method according will be described below with reference to an example where the first target analysis tool is in a second A-form that uses aptamers.

In the second A-form, the first binding substances in the labeled first binding substances are aptamers. The aptamers are the same as described for the first A-form, for example.

The second binding substances in the immobilized second binding substances are not limited as long as they can bind to the aptamers, and may be, for example, nucleic acid molecules complementary to the aptamers. The nucleic acid molecules complementary to the aptamers are the same as described for the first A-form, for example.

The labeling substances in the labeled first binding substances preferably are catalytic nucleic acid molecules exhibiting a catalytic function or an enzyme, for example. The catalytic nucleic acid molecules and the enzyme are the same as described for the first A-form, for example.

The carriers in the immobilized second binding substances may be beads, or an inner wall of the second chamber may serve as the carrier of the immobilized second binding substances, for example. The beads are the same as described for the first A-form, for example. When the carriers are the beads, the amount of the second binding substances to be immobilized on the carriers (beads) is not particularly limited, and may be, for example, from 0.1 fmol to 100 pmol, 1 fmol to 10 pmol, or 10 fmol to 1 pmol per mm² surface area of the bead. When the inner wall of the second chamber is the carrier, the amount of the second binding substances to be immobilized on the carrier (inner wall) is not particularly limited, and may be, for example, from 0.1 fmol to 100 pmol, 1 fmol to 10 pmol, or 10 fmol to 1 pmol per mm² area of the inner wall of the second chamber.

In the second A-form, as described above, aptamers can be used as the first binding substances, complementary nucleic acid molecules can be used as the second binding substances, and catalytic nucleic acid molecules can be used as the labeling substances. Thus, the target analysis tool in the second A-form is thermostable and can be stored more easily, for example. It is also possible to use an enzyme such as luciferase, alkaline phosphatase, or peroxidase as the labeling substances, for example. Thus, it is possible to analyze a target with high sensitivity, for example.

The first partition wall between the first chamber and the second chamber and the second partition wall between the second chamber and the third chamber are the same as described for the first A-form.

It is preferable that the second chamber further contains, as the second reagent, adsorbent carriers that adsorb at least one of proteins and lipids. The adsorbent carriers are the same as described for the first A-form, for example.

It is preferable that the third chamber further contains a substrate for the catalytic function of the catalytic nucleic acid molecules or the enzyme, for example. Examples of the substrate include substrates for peroxidases and substrates for enzymes such as luciferase and alkaline phosphatase.

An example of a target analysis method using a target analysis tool in the second A-form will be described specifically with reference to drawings. Unless otherwise stated, the above description regarding the first A-form also applies to the second A-form.

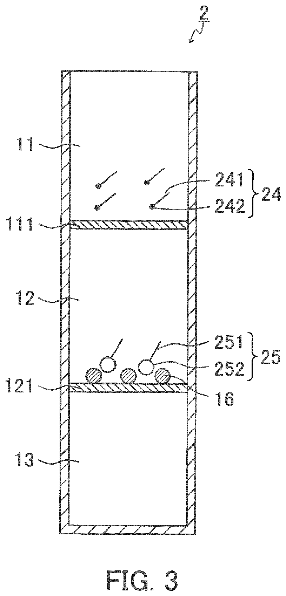

FIG. 3 schematically shows a target analysis tool in the second A-form and a target analysis method using this target analysis tool. In FIG. 3, the same components as those in FIG. 1 are given the same reference numerals. A target analysis tool 2 includes a first chamber 11, a second chamber 12, and a third chamber 13. In the first chamber 11, labeled aptamers 24 obtained by adding a DNAzyme 242 to aptamers 241 are disposed. In the second chamber 12, immobilized complementary strands 25, which are obtained by immobilizing complementary strands 251 complementary to the aptamers 241 on beads 252, and silica beads 16 are disposed. A first partition wall 111 is provided between the first chamber 11 and the second chamber 12. A porous second partition wall 121 is provided between the second chamber 12 and the third chamber 13.

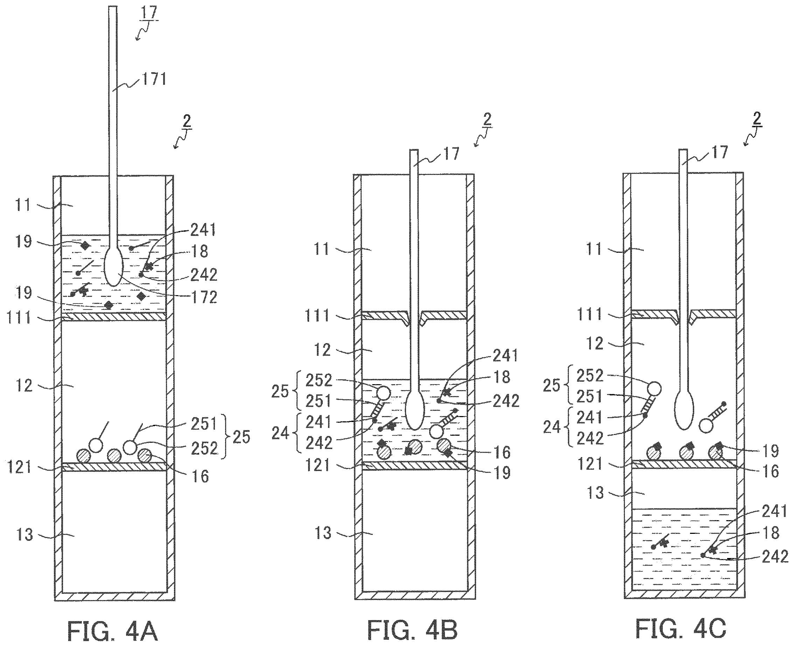

Next, an analysis method using the target analysis tool 2 shown in FIG. 3 will be described with reference to FIGs. 4A to 4C. FIGs. 4A to 4C schematically show the method of using the target analysis tool 2.

First, the specimen holder 17 with the specimen being held in the holding section 172 is inserted to the first chamber 11 of the target analysis tool 2, thereby binding the target 18 in the specimen to the aptamers 241 in the labeled aptamers 24 in the first chamber 11. Conditions of the treatment for binding them are not particularly limited, and may be as follows, for example: the treatment temperature is from 4°C to 37°C and the treatment time is from 10 seconds to 30 minutes.

Next, the specimen holder 17 breaks the first partition wall 111, whereby the contents in the first chamber 11 is introduced to the second chamber 12. Then, among the labeled aptamers 24, those not bound to the target 18 are caused to bind to the immobilized complementary strands 25. Conditions of the treatment for binding them are not particularly limited, and may be as follows, for example: the treatment temperature is from 4°C to 37°C and the treatment time is from 10 seconds to 30 minutes.

The second partition wall 121 is a porous partition wall through which the immobilized complementary strands 25 do not pass and the labeled aptamers 24 pass. Accordingly, among the labeled aptamers 24, only those not bound to the immobilized complementary strands 25 pass through the second partition wall 121 to be introduced to the third chamber 13. In the second chamber 12, the silica beads 16 are disposed. Accordingly, for example, contaminants 19 such as proteins and lipids derived from the specimen are adsorbed onto the silica beads 16, whereby the contaminants 19 are prevented from being introduced to the third chamber 13. Then, in the third chamber 13, by subjecting the unbound labeled aptamers 24 to measurement of the catalytic function of the DNAzyme 242, the target in the specimen can be analyzed indirectly. The method for measuring the catalytic function of the DNAzyme 242 can be determined as appropriate depending on the type of the DNAzyme 242.

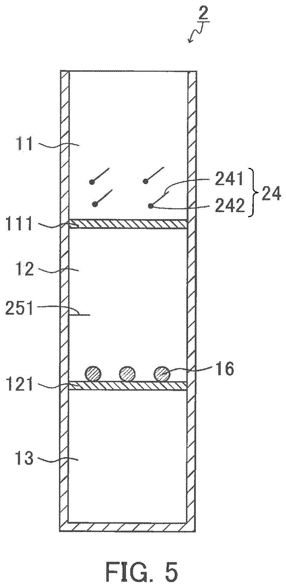

FIG. 5 schematically shows another example of the target analysis tool in the second A-form. Unless otherwise stated, FIG. 5 is the same as FIG. 3. In FIG. 5, complementary strands 251 are immobilized on the inner wall of a second chamber 12. Since the complementary strands 251 are immobilized on the inner wall of the second chamber 12, labeled aptamers 24 bound to the complementary strands 251 immobilized on the inner wall of the second chamber 12 remain in the second chamber 12 without moving to a third chamber 13 from the second chamber 12.

The second target analysis tool may further include a pretreatment chamber, for example. The second target analysis tool may be configured so that the pretreatment chamber is disposed continuously with the first chamber on the side opposite to the second chamber. That is, it is preferable that, in the second target analysis tool, the pretreatment chamber, the first chamber, the second chamber, and the third chamber are disposed in this order.

The pretreatment chamber may be configured so that, for example, the specimen holder can be inserted from the outside to the inside of the pretreatment chamber, and the pretreatment chamber contains an extractant for extracting a component(s) from the specimen. The extractant is not particularly limited, and can be selected as appropriate depending on the type of the specimen to be subjected to the analysis, the type of a component to be analyzed, etc.

The second target analysis tool is configured so that, for example, a partition wall is provided between the pretreatment chamber and the first chamber, and the partition wall is a partition wall that is broken upon contact with the tip of the specimen holder inserted into the pretreatment chamber. The partition wall to be broken is, for example, the same as described above, and may be aluminum foil or the like, for example.

The second target analysis tool may be configured so that, for example, the pretreatment chamber has a cover for an opening of the pretreatment chamber on the side opposite to the partition wall with the first chamber, and the specimen holder can be inserted from an outside of the cover to an inside of the pretreatment chamber. The cover preferably is a partition wall that is broken upon contact with the tip of the specimen holder. Examples of the partition wall include those given above as examples of the partition wall.

The second target analysis tool may include an outer cylinder and an inner cylinder, for example. The above descriptions regarding the outer cylinder and the inner cylinder also apply to the outer cylinder and the inner cylinder in the second target analysis tool, for example.

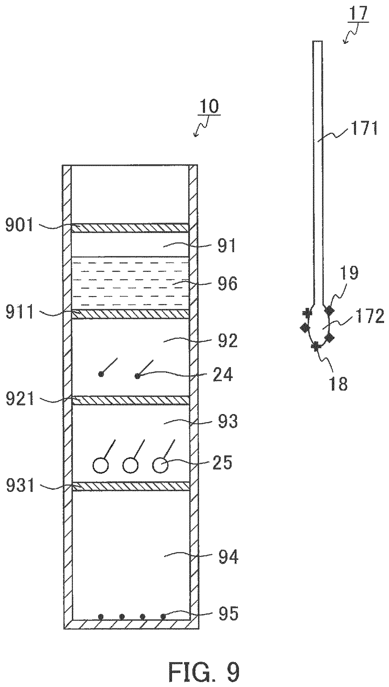

The second target analysis tool and the second target analysis method will be described with reference to FIG. 9, taking an example where the second target analysis tool is in a second B-form further including the pretreatment chamber. This second B-form is a variation of the second A-form. As in the second A-form, aptamers are used as the first binding substances, and nucleic acid molecules complementary to the aptamers are used as the second binding substances in the second B-form. Unless otherwise stated, the above description regarding the second A-form also applies to the second B-form.

FIG. 9 schematically shows a target analysis tool in the second B-form. In FIG. 9, the same components as those in FIGs. 3 and 4 are given the same reference numerals. A target analysis tool 10 includes a pretreatment chamber 91, a first chamber 92, a second chamber 93, and a third chamber 94. In the first chamber 92, labeled aptamers 24 obtained by adding a DNAzyme to aptamers are disposed. The labeled aptamers 24 preferably are in a dry state, because the degradation of the labeled aptamers 24 can be prevented more reliably, for example. The second chamber 93 is filled with immobilized complementary strands 25 obtained by immobilizing complementary strands to the aptamers on beads (the beads may be formed of a resin, for example). In the third chamber 94, a substrate 95 for the DNAzyme is disposed. The substrate 9 preferably is in a dry state. The pretreatment chamber 91 has a cover 901 for covering the upper opening of the pretreatment chamber 91. A partition wall 911 is provided between the pretreatment chamber 91 and the first chamber 92. A porous first partition wall 921 is provided between the first chamber 92 and the second chamber 93. A porous second partition wall 931 is provided between the second chamber 93 and the third chamber 94. The pretreatment chamber 91 contains an extractant 96 introduced thereto.

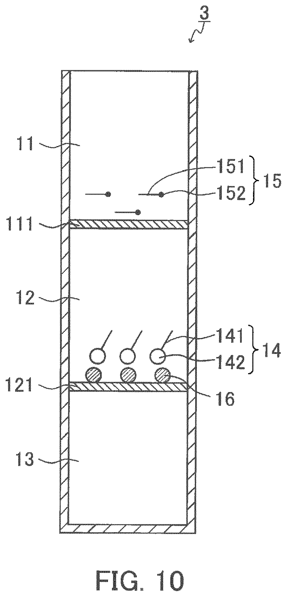

Next, an analysis method using the target analysis tool 9 shown in FIG. 10 will be described.

First, the specimen holder 17 holding a specimen is inserted to the target analysis tool 10 so that the cover 901 of the pretreatment chamber 91 and the partition wall 911 are pierced through with the tip of the specimen holder 17. This allows the extractant 96 in the pretreatment chamber 91 to be introduced to the first chamber 92. Then, in the first chamber 92, the target 18 in the specimen binds to the labeled aptamers 24. At this time, it is preferable to shake the target analysis tool to mix the target 18 and the labeled aptamers 24.

In the specimen holder 17, the first partition wall 921 is porous, so that the contents in the first chamber 92 are introduced to the second chamber 93 through the pores of the first partition wall 921. Then, among the labeled aptamers 24, those not bound to the target 18 bind to the immobilized complementary strands 25.

The second partition wall 931 is a porous partition wall through which the immobilized complementary strands 25 do not pass and the labeled aptamers 24 pass. Accordingly, among the labeled aptamers 24, only those not bound to the immobilized complementary strands 25 pass through the second partition wall 931 to be introduced to the third chamber 94. Then, in the third chamber 94, by subjecting the unbound labeled aptamers 24 to measurement of the catalytic function of the DNAzyme 242, the target in the specimen can be analyzed indirectly. The method for measuring the catalytic function of the DNAzyme 242 and the substrate 95 disposed in the third chamber 94 can be determined as appropriate depending on the type of the DNAzyme 242, for example.

According to the second B-form, analysis can be carried out easily by merely pushing the specimen holder 17 once and optionally shaking the specimen holder 17, for example. In the present example, the target can be analyzed in the same manner when an enzyme is used instead of the DNAzyme, for example.

### [Third Target Analysis Tool and Third Target Analysis Method]

The third target analysis tool and the third target analysis method will be described specifically below. Unless otherwise stated, descriptions regarding the first, second, and fourth target analysis tools and the first, second, and fourth target analysis methods also apply to the third target analysis tool and the third target analysis method.

The third target analysis method is, as described above, a target analysis tool including: a first chamber; a second chamber; and a third chamber, wherein the first chamber, the second chamber, and the third chamber are disposed continuously in this order, the first chamber contains, as a first reagent, a labeled second binding substance obtained by binding a labeling substance to a second binding substance that binds to a first binding substance that binds to a target, the second chamber contains, as a second reagent, an immobilized first binding substance obtained by immobilizing the first binding substance on a carrier, the third chamber is a detection section in which the labeled second binding substance is detected, a first partition wall is provided between the first chamber and the second chamber, a second partition wall is provided between the second chamber and the third chamber, the first chamber is configured so that a specimen holder having a specimen can be inserted from an outside of the first chamber to an inside of the first chamber, the first partition wall is a partition wall that is broken upon contact with a tip of the specimen holder inserted into the first chamber or a porous partition wall through which contents in the first chamber can pass to enter the second chamber, and the second partition wall is a porous partition wall through which the immobilized first binding substance cannot pass and the labeled second binding substance can pass.

The third target analysis method is, as described above, a target analysis method using the third target analysis tool, including the steps of: inserting a specimen holder holding a specimen into the first chamber of the target analysis tool; binding a target in the specimen to the immobilized first binding substance as the second reagent and also binding an unbound immobilized first binding substance not bound to the target to the labeled second binding substance as the first reagent by causing a mixture of the specimen and the first reagent to be in contact with the second reagent in the second chamber; introducing an unbound labeled second binding substance to the third chamber through the second partition wall between the second chamber and the third chamber; and detecting the labeling substance in the labeled second binding substance in the third chamber.

As described herein, first, in the first chamber, for example, a target in a specimen is mixed with the labeled second binding substances as the first reagent. Then, the mixture of the specimen and the first reagent in the first chamber is introduced to the second chamber. In the second chamber, the target in the specimen binds to the immobilized first binding substances as the second reagent, and the immobilized first binding substances not bound to the target bind to the labeled second binding substances as the first reagent. The second partition wall between the second chamber and the third chamber is a porous partition wall through which the immobilized first binding substances cannot pass and the labeled second binding substances can pass. Accordingly, the immobilized first binding substances do not pass through the partition wall and thus remain in the second chamber. That is, the labeled second binding substances bound to the immobilized first binding substances remain in the second chamber without moving to the third chamber. On the other hand, the labeled second binding substances that are in a free state without being bound to the immobilized first binding substances are introduced to the third chamber through the partition wall. The target analysis tool can contain a known amount of the labeled second binding substances, for example. Accordingly, the amount of the labeled second binding substances not bound to the immobilized first binding substances indirectly corresponds to the amount of the target in the specimen. Thus, by detecting the unbound labeled second binding substances introduced to the third chamber, the presence or absence of the target or the amount of the target in the specimen can be analyzed indirectly.

As described herein, the first partition wall may be a partition wall that is broken upon contact with the tip of the specimen holder inserted into the first chamber or a porous partition wall through which contents in the first chamber can pass to enter the second chamber, for example. In the former case, the contents in the first chamber can move to the second chamber by breaking the first partition wall, for example. In the latter case, the contents in the first chamber can move to the second chamber thorough the porous partition wall. In the former case, it is possible to use a member that can be broken in the above-described manner. In the latter case, it is possible to use a membrane having pores through which the contents in the first chamber can pass, for example.

The term "analysis" as used may mean, for example, qualitative analysis to determine the presence or absence of a target or quantitative analysis to determine the amount of a target.

As described above, the first binding substances that bind to a target to be used are not limited to a particular type of binding substances, as long as they bind to the target, for example. Specifically, the first binding substances may be aptamers or antibodies, for example. The third target analysis tool and the third target analysis method will be described below with reference to an example where the third target analysis tool is in a third A-form that uses aptamers.

In the third A-form, the first binding substances in the immobilized first binding substances are aptamers. The aptamers are the same as described for the first A-form, for example.

The second binding substances in the labeled second binding substances are not limited as long as they can bind to the aptamers, and may be, for example, nucleic acid molecules complementary to the aptamers. The nucleic acid molecules complementary to the aptamers are the same as described for the first A-form, for example.

The labeling substances in the labeled second binding substances preferably are catalytic nucleic acid molecules exhibiting a catalytic function or an enzyme, for example. The catalytic nucleic acid molecules and the enzyme are the same as described for the first A-form, for example.

The carriers in the immobilized first binding substances may be beads, or an inner wall of the second chamber may serve as the carrier of the immobilized second binding substances, for example. The beads are the same as described for the first A-form, for example. When the carriers are the beads, the amount of the aptamers to be immobilized on the carriers (beads) is not particularly limited, and may be, for example, from 0.1 fmol to 100 pmol, 1 fmol to 10 pmol, or 10 fmol to 1 pmol per mm² surface area of the bead. When the inner wall of the second chamber is the carrier, the amount of the aptamers to be immobilized on the carrier (inner wall) is not particularly limited, and may be, for example, from 0.1 fmol to 100 pmol, 1 fmol to 10 pmol, or 10 fmol to 1 pmol per mm² area of the inner wall of the second chamber.

In the third A-form, as described above, aptamers can be used as the first binding substances, complementary nucleic acid molecules can be used as the second binding substances, and catalytic nucleic acid molecules can be used as the labeling substances. Thus, the target analysis tool in the third A-form is thermostable and can be stored more easily, for example. It is also possible to use an enzyme such as luciferase or alkaline phosphatase as the labeling substances, for example. Thus, it is possible to analyze a target with high sensitivity, for example.

The first partition wall between the first chamber and the second chamber and the second partition wall between the second chamber and the third chamber are the same as described for the first A-form.

It is preferable that the second chamber further contains, as the second reagent, adsorbent carriers that adsorb at least one of proteins and lipids. The adsorbent carriers are the same as described for the first A-form, for example.

It is preferable that the third chamber further contains a substrate for the catalytic function of the catalytic nucleic acid molecules or the enzyme, for example. Examples of the substrate include substrates for peroxidases and substrates for enzymes such as luciferase and alkaline phosphatase.

An example of a target analysis method using a target analysis tool in the third A-form will be described specifically with reference to drawings. Unless otherwise stated, the above descriptions regarding the first A and 2A-form also apply to the third A-form.

FIG. 10 schematically shows a target analysis tool in the third A-form and a target analysis method using the same. In FIG. 10, the same components as those in FIG. 1 are given the same reference numerals. A target analysis tool 3 includes a first chamber 11, a second chamber 12, and a third chamber 13. In the first chamber 11, labeled complementary strands 15 obtained by adding a DNAzyme 152 to complementary strands 151 complementary to aptamers 141 are disposed. In the second chamber 12, immobilized aptamers 14, which are obtained by immobilizing the aptamers 141 on beads 142, and silica beads 16 are disposed. A first partition wall 111 is provided between the first chamber 11 and the second chamber 12. A porous second partition wall 121 is provided between the second chamber 12 and the third chamber 13.

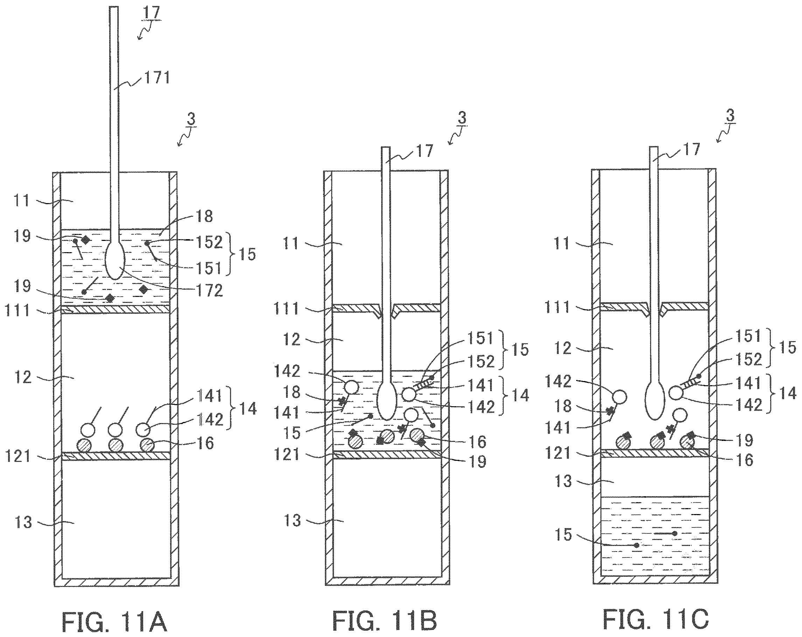

Next, an analysis method using the target analysis tool 3 shown in FIG. 10 will be described with reference to FIGs. 11A to 11C. FIGs. 11A to 11C schematically show the method of using the target analysis tool 3.

First, the specimen holder 17 with the specimen being held in the holding section 172 is inserted to the first chamber 11 of the target analysis tool 3, thereby mixing the target 18 in the specimen with the labeled complementary strands 15 in the first chamber 11. Conditions of the treatment for mixing them are not particularly limited, and may be as follows, for example: the treatment temperature is from 4°C to 37°C and the treatment time is from 10 seconds to 30 minutes.

Next, the specimen holder 17 breaks the first partition wall 111, whereby the contents in the first chamber 11 are introduced to the second chamber 12. Then, the target 18 in the contents is caused to bind to the immobilized aptamers 14, and also, among the immobilized aptamers 14, those not bound to the target 18 are caused to bind to the labeled complementary strands 15. Conditions of the treatment for binding them are not particularly limited, and may be as follows, for example: the treatment temperature is from 4°C to 37°C and the treatment time is from 10 seconds to 30 minutes.

The second partition wall 121 is a porous partition wall through which the immobilized aptamers 14 do not pass and the labeled complementary strands 15 pass. Accordingly, among the labeled complementary strands 15, only those not bound to the immobilized aptamers 14 pass through the second partition wall 121 to be introduced to the third chamber 13. In the second chamber 12, the silica beads 16 are disposed. Accordingly, for example, contaminants 19 such as proteins and lipids derived from the specimen are adsorbed onto the silica beads 16, whereby the contaminants 19 are prevented from being introduced to the third chamber 13. Then, in the third chamber 13, by subjecting the labeled complementary strands 15 to measurement of the catalytic function of the DNAzyme 152, the target in the specimen can be analyzed indirectly. The method for measuring the catalytic function of the DNAzyme 152 can be determined as appropriate depending on the type of the DNAzyme 152.

The third target analysis tool may further include a pretreatment chamber, for example. The above description regarding the pretreatment chamber also applies to the pretreatment chamber in the third target analysis tool, for example. The third target analysis tool may include an outer cylinder and an inner cylinder, for example. The above descriptions regarding the outer cylinder and the inner cylinder also apply to the outer cylinder and the inner cylinder in the third target analysis tool, for example.

### [Fourth Target Analysis Tool and Fourth Target Analysis Method]

The fourth target analysis tool and the fourth target analysis method of the present invention will be described specifically below. Unless otherwise stated, descriptions regarding the first to third target analysis tools and the first to third target analysis methods also apply to the fourth target analysis tool and the fourth target analysis method.

The fourth target analysis method according to the present invention is, as described above, a target analysis tool including: a first chamber; a second chamber; and a third chamber, wherein the first chamber, the second chamber, and the third chamber are disposed continuously in this order, the first chamber contains, as a first reagent, an immobilized second binding substance obtained by immobilizing, on a carrier, a second binding substance that binds to a first binding substance that binds to a target, the second chamber contains, as a second reagent, a labeled first binding substance obtained by binding a labeling substance to the first binding substance, the third chamber is a detection section in which the labeled first binding substance is detected, a first partition wall is provided between the first chamber and the second chamber, a second partition wall is provided between the second chamber and the third chamber, the first chamber is configured so that a specimen holder having a specimen can be inserted from an outside of the first chamber to an inside of the first chamber, the first partition wall is a partition wall that is broken upon contact with a tip of the specimen holder inserted into the first chamber, and the second partition wall is a porous partition wall through which the immobilized second binding substance cannot pass and the labeled first binding substance not bound to the immobilized second binding substance can pass.

The fourth target analysis method according to the present invention is, as described above, a target analysis method using the fourth target analysis tool according to the present invention, including the steps of: introducing a specimen and the first reagent to the second chamber by inserting a specimen holder holding the specimen into the first chamber of the target analysis tool and then bringing the specimen holder into contact with the first partition wall between the first chamber and the second chamber; binding a target in the specimen to the labeled first binding substance as the second reagent to form a first conjugate and also binding an unbound labeled first binding substance not bound to the target to the immobilized second binding substance as the first reagent by causing the specimen and the first reagent to be in contact with the second reagent in the second chamber; introducing the first conjugate to the third chamber through the second partition wall between the second chamber and the third chamber; and detecting the labeled first binding substance in the first conjugate in the third chamber.

According to the present invention, first, in the first chamber, for example, a target in a specimen is mixed with the immobilized second binding substances as the first reagent. Then, the mixture of the specimen and the first reagent in the first chamber is introduced to the second chamber. In the second chamber, the target in the specimen binds to the labeled first binding substances as the second reagent, and the labeled first binding substances not bound to the target bind to the immobilized second binding substances as the first reagent. The second partition wall between the second chamber and the third chamber is a porous partition wall through which the immobilized second binding substances cannot pass and the labeled first binding substances not bound to the immobilized second binding substance can pass. Accordingly, the immobilized second binding substances do not pass through the partition wall and thus remain in the second chamber. That is, the labeled first binding substances bound to the immobilized second binding substances remain in the second chamber without moving to the third chamber. On the other hand, the labeled first binding substances that are in a free state without being bound to the immobilized second binding substances, i.e., the labeled first binding substances forming the first conjugates, are introduced to the third chamber through the partition wall. The target analysis tool of the present invention can contain a known amount of the labeled first binding substances, for example. Accordingly, the amount of the labeled first binding substances not bound to the immobilized second binding substances indirectly corresponds to the amount of the target in the specimen. Thus, by detecting the unbound labeled first binding substances introduced to the third chamber, the presence or absence of the target or the amount of the target in the specimen can be analyzed indirectly.

The term "analysis" as used in the present invention may mean, for example, qualitative analysis to determine the presence or absence of a target or quantitative analysis to determine the amount of a target.

As described above, the first binding substances that bind to a target to be used are not limited to a particular type of binding substances, as long as they bind to the target, for example. The first binding substances according to the invention are aptamers. The fourth target analysis tool and the fourth target analysis method according to the present invention will be described below with reference to an example where the fourth target analysis tool is in a fourth A-form that uses aptamers.

In the fourth A-form of the present invention, the first binding substances in the labeled first binding substances are aptamers. The aptamers are the same as described for the first A-form, for example.

The second binding substances in the immobilized second binding substances are nucleic acid molecules complementary to the aptamers. The nucleic acid molecules complementary to the aptamers are the same as described for the first A-form, for example.

The labeling substances in the labeled first binding substances preferably are catalytic nucleic acid molecules exhibiting a catalytic function or an enzyme, for example. The catalytic nucleic acid molecules and the enzyme are the same as described for the first A-form, for example.

The carriers in the immobilized second binding substances may be beads, for example. The beads are the same as described for the first A-form, for example. When the carriers are the beads, the amount of the aptamers to be immobilized on the carriers (beads) is not particularly limited, and may be, for example, from 0.1 fmol to 100 pmol, 1 fmol to 10 pmol, or 10 fmol to 1 pmol per mm² surface area of the bead.

In the fourthA-form of the present invention, as described above, aptamers are used as the first binding substances, complementary nucleic acid molecules are used as the second binding substances, and catalytic nucleic acid molecules can be used as the labeling substances. Thus, the target analysis tool in the fourth A-form is thermostable and can be stored more easily, for example. It is also possible to use an enzyme such as luciferase, alkaline phosphatase, or peroxidase as the labeling substances, for example. Thus, it is possible to analyze a target with high sensitivity, for example.

The first partition wall between the first chamber and the second chamber and the second partition wall between the second chamber and the third chamber are the same as described for the first A-form.

It is preferable that the second chamber further contains, as the second reagent, adsorbent carriers that adsorb at least one of proteins and lipids. The adsorbent carriers are the same as described for the first A-form, for example.

It is preferable that the third chamber further contains a substrate for the catalytic function of the catalytic nucleic acid molecules or the enzyme, for example. Examples of the substrate include substrates for peroxidases and substrates for enzymes such as luciferase and alkaline phosphatase.

An example of a target analysis method using a target analysis tool in the fourth A-form of the present invention will be described specifically with reference to drawings. It is to be noted, however, that the present invention is not limited to this illustrative example. Unless otherwise stated, the above descriptions regarding the first to third A-forms also apply to the fourth A-form of the present invention.

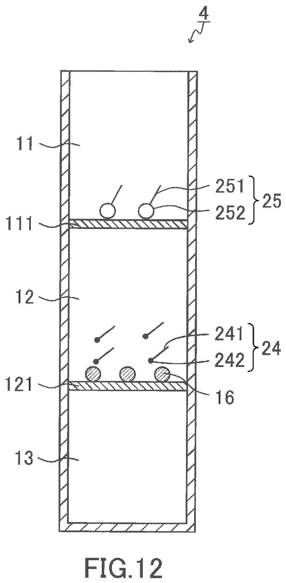

FIG. 12 schematically shows a target analysis tool in the fourth A-form and a target analysis method using the same. In FIG. 12, the same components as those in FIG. 3 are given the same reference numerals. A target analysis tool 4 includes a first chamber 11, a second chamber 12, and a third chamber 13. In the first chamber 11, immobilized complementary strands 25 obtained by immobilizing complementary strands 251 complementary to aptamers 241 on beads 252 are disposed. In the second chamber 12, labeled aptamers 24, which are obtained by adding a DNAzyme 242 to the aptamers 241, and silica beads 16 are disposed. A first partition wall 111 is provided between the first chamber 11 and the second chamber 12. A porous second partition wall 121 is provided between the second chamber 12 and the third chamber 13. A first partition wall 111 is provided between the first chamber 11 and the second chamber 12. A porous second partition wall 121 is provided between the second chamber 12 and the third chamber 13.

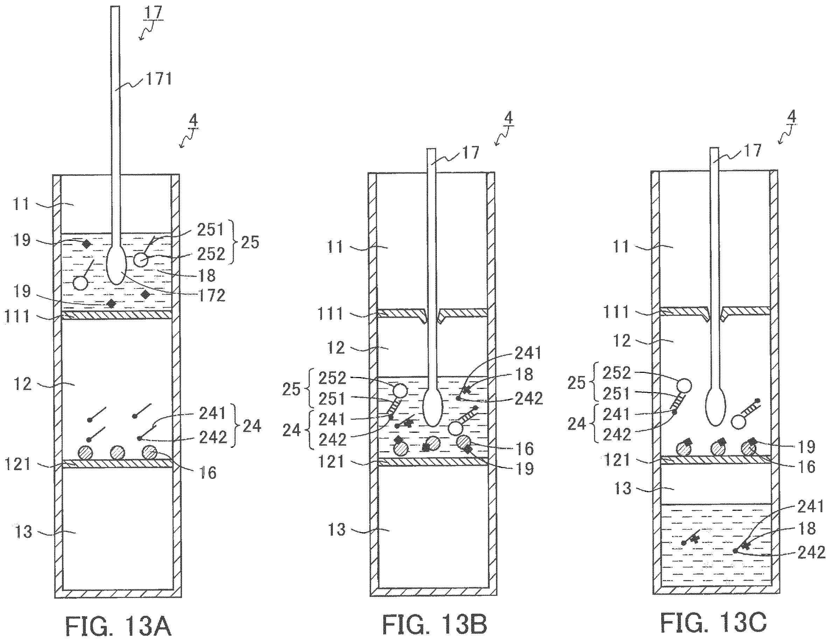

Next, an analysis method using the target analysis tool 4 shown in FIG. 12 will be described with reference to FIGs. 13A to 13C. FIGs. 13A to 13C schematically show the method of using the target analysis tool 4.

First, the specimen holder 17 with the specimen being held in the holding section 172 is inserted to the first chamber 11 of the target analysis tool 4, thereby mixing the target 18 in the specimen with the immobilized complementary strands 25 in the first chamber 11. Conditions of the treatment for mixing them are not particularly limited, and may be as follows, for example: the treatment temperature is from 4°C to 37°C and the treatment time is from 10 seconds to 30 minutes.

Next, the specimen holder 17 breaks the first partition wall 111, whereby the contents in the first chamber 11 are introduced to the second chamber 12. Then, the target 18 in the contents are caused to bind to the labeled aptamers 24, and also, among the labeled aptamers 24, those not bound to the target 18 are caused to bind to the immobilized complementary strands 25. Conditions of the treatment for binding them are not particularly limited, and may be as follows, for example: the treatment temperature is from 4°C to 37°C and the treatment time is from 10 seconds to 30 minutes.

The second partition wall 121 is a porous partition wall through which the immobilized complementary strands 25 do not pass and the labeled aptamers 24 pass. Accordingly, among the labeled aptamers 24, only those not bound to the immobilized complementary strands 25 pass through the second partition wall 121 to be introduced to the third chamber 13. In the second chamber 12, the silica beads 16 are disposed. Accordingly, for example, contaminants 19 such as proteins and lipids derived from the specimen are adsorbed onto the silica beads 16, whereby the contaminants 19 are prevented from being introduced to the third chamber 13. Then, in the third chamber 13, by subjecting the labeled aptamers 24 to measurement of the catalytic function of the DNAzyme 242, the target in the specimen can be analyzed indirectly. The method for measuring the catalytic function of the DNAzyme 242 can be determined as appropriate depending on the type of the DNAzyme 242.

The fourth target analysis tool of the present invention may further include a pretreatment chamber, for example. The above description regarding the pretreatment chamber also applies to the pretreatment chamber in the fourth target analysis tool, for example. The fourth target analysis tool of the present invention may include an outer cylinder and an inner cylinder, for example. The above descriptions regarding the outer cylinder and the inner cylinder also apply to the outer cylinder and the inner cylinder in the fourth target analysis tool, for example.

### Examples

Examples 1-4 and 6 are for comparison reasons only.

### (Example 1)

The target analysis tool in the first A-form is configured so that, for example, the silica beads 16 are disposed in the second chamber 12 as shown in FIGs. 1 and 2. With this configuration, contaminants such as proteins are adsorbed onto the silica beads 16, whereby the contaminants can be prevented from being introduced to the third chamber 13. Thus, the present example examined whether a contaminant protein is removed by the silica beads 16.

### (1) Examination 1 on protein removal

As a buffer solution, 2 × buffer (80 mmol/l HEPES, 250 mmol/l NaCl, 10 mmol/l KCl, 2 mmol/l MgCl₂, and 0.1% Tween 20) was used. Human α-thrombin was used as the contaminant protein. The human α-thrombin was added to 50% glycerol at a concentration of 200 ppm to prepare a thrombin solution. Silica beads (trade name: Silicon dioxide, SIGMA) were added to distilled water at a concentration of 1 g/ml to prepare a silica bead solution.

The thrombin solution was added to 25 µl of the buffer solution so that the final concentration of the human α-thrombin was 100 ppm. 5 µl of the silica beads solution was further added thereto, so that, in the mixed solution, the human α-thrombin was adsorbed onto the silica beads. Then, the mixed solution was centrifuged (11,000 G, 1 minute), and a liquid fraction was collected by removing the silica beads. The human α-thrombin in the liquid fraction was detected. The human α-thrombin was detected by measuring the absorbance of the liquid fraction at wavelengths from 230 to 330 nm using a spectrophotometer (trade name: NanoDrop, Thermo SCIENTIFIC, the same applies hereinafter). As a control, 25 µl of the thrombin solution was added to 25 µl of the buffer solution to prepare a solution containing 100 ppm of the human α-thrombin, and the absorbance of this solution was measured.

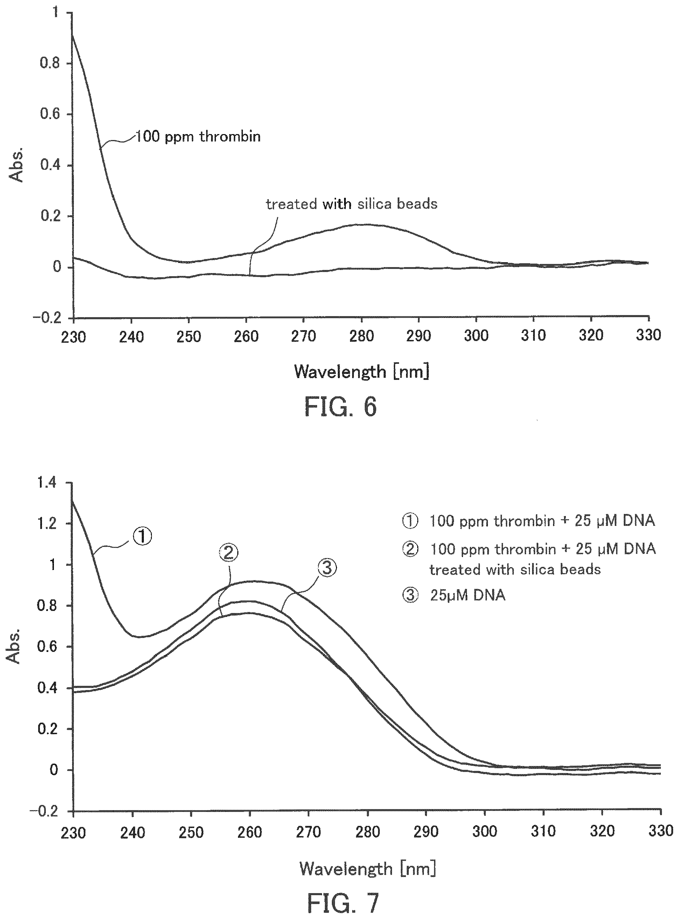

The results thereof are shown in FIG. 6. FIG. 6 is a graph showing the absorbance of the liquid fraction. FIG. 6 shows the result obtained regarding the liquid fraction treated with the silica beads, together with the result obtained regarding the 100 ppm human α-thrombin solution. As can be seen in FIG. 6, the 100 ppm human α-thrombin solution showed absorption at around 280 nm. In contrast, the liquid fraction treated with the silica beads did not show absorption at around 280 nm. These results demonstrate that the human α-thrombin was adsorbed onto the silica beads and thus removed.

### (2) Examination 2 on protein removal

In the above item (1), it was found that a contaminant protein can be removed by the silica beads. On the other hand, there is a case where nucleic acid molecules such as aptamers are used as first binding substances that bind to a target, and nucleic acid molecules complementary to the aptamers are used as second binding substances that bind to the first binding substances. Thus, the present examination was conducted in order to demonstrate that the nucleic acid molecules are not removed by the silica beads.

As the nucleic acid molecule, a DNA molecule having the following sequence was used.
DNA molecule (SEQ ID NO: 1)
5'-AAAAACCAACCACACCAACC-3'

25 µl of the thrombin solution was added to 25 µl of the buffer solution so that the final concentration of the human α-thrombin was 100 ppm. Next, the DNA molecules were added thereto so that the final concentration of the DNA molecules was 25 µmol/l. 5 µl of the silica beads solution was further added thereto, so that, in the mixed solution, the human α-thrombin was adsorbed onto the silica beads. Then, the mixed solution was centrifuged (11,000 G, 1 minute), and a liquid fraction was collected by removing the silica beads. The absorbance of the liquid fraction was measured at wavelengths from 230 to 330 nm using the spectrophotometer. As Controls 1 and 2, the absorbance measurement was performed on the following solutions, respectively: Solution 1 containing 100 ppm of the human α-thrombin and 25 µmol/l of the DNA molecules, prepared by adding the thrombin solution and the DNA molecules to 25 µl of the buffer solution; and Solution 2 prepared by adding the DNA molecules to 50 µl of the buffer solution so that the final concentration of the DNA molecules was 25 µmol/l.

The results thereof are shown in FIG. 7. FIG. 7 is a graph showing the absorbance of the liquid fraction. FIG. 7 shows the result obtained regarding the liquid fraction treated with the 100 ppm silica beads, together with the results obtained regarding Controls 1 and 2. As can be seen in FIG. 7, Control 1 (100 ppm thrombin + 25 µmol/l DNA), which contained a protein (thrombin) and a nucleic acid (DNA molecules), showed the highest absorbance over a broad wavelength region. In contrast, in the liquid fraction collected after treating the mixed solution containing the protein and the nucleic acid with the silica beads, only the protein had been removed by the silica beads. Thus, the absorption curve of the liquid fraction overlapped with the absorption curve of Control 2 containing only the nucleic acid (25 µmol/l DNA), and the liquid fraction showed absorption at 260 nm. This demonstrates that DNA in the liquid fraction can be detected.

### (Example 2)

The target analysis tool in the first A-form is configured so that, for example, as shown in FIG. 2, the aptamers 141 in the immobilized aptamers 14 are caused to bind to the complementary strands 151 complementary to the labeled complementary strands 15 in the second chamber 12, and among the labeled complementary strands 15, only those not bound to the immobilized aptamers 14 are introduced to the third chamber. Thus, the present example carried out the treatments in the presence of a target protein, and measured the amount of complementary strands collected finally.

### (1) Immobilized aptamers

Immobilized aptamers were prepared by binding aptamers to beads. As the beads, magnetic beads with having streptavidin added thereto (trade name: Dynabeads MyOne streptavidin C1, Invitrogen) were used. As the aptamers, thrombin aptamers (TA-3bio) that bind to thrombin were used. The thrombin aptamer is represented by the following sequence and has biotin and five thymine bases added at the 3' end. The immobilized aptamers were prepared by immobilizing 1000 pmol of the thrombin aptamers per 2 mg of the magnetic beads.
Thrombin aptamer (SEQ ID NO: 2)
5'-GGTTGGTGTGGTTGGTTTTT-3'

### (2) Complementary strands

Complementary strands complementary to the thrombin aptamers were prepared.
Complementary strand (SEQ ID NO: 3)
5'-AAAAACCAACCACACCAACC-3'

### (3) Method

The immobilized aptamers were added to the buffer solution to prepare an immobilized aptamer solution. The complementary strands were added to distilled water to prepare a complementary strand solution. Silica beads (trade name: Silicon dioxide, Sigma Chemical Co.) were added to distilled water at a concentration of 1g/ml to prepare a silica bead solution. Human α-thrombin was used as a target protein. The human α-thrombin was added to 50% glycerol to prepare a thrombin solution.

100 µl of the immobilized aptamer solution was added to 25 µl of the buffer solution so that the amount of aptamers derived from the immobilized aptamers was 1000 pmol. 25 µl of the thrombin solution was added thereto so as to achieve predetermined final concentrations of the human α-thrombin (0, 1, 3, 10, 30, and 100 ppm). Each of the resultant mixed solutions was stirred at room temperature (around 25°C) for 10 minutes, thereby causing the immobilized aptamers to bind to the human α-thrombin as the target protein. To this mixed solution, 100 µl of the complementary strand solution (the complementary strands: 1000 pmol) was further added so that the resultant mixed solution contained the same amount of the complementary strands (25 µmol/l) as the aptamers derived from the immobilized aptamers. The resultant mixed solution was stirred at room temperature for 10 minutes, thereby causing the complementary strands to bind to the aptamers in the immobilized aptamers not bound to the target protein. Then, 50 µl of this mixed solution was collected as a sample, and mixed with 5 µl of the silica beads solution. Then, the mixed solution was centrifuged (11,000 G, 1 minute), and a liquid fraction was collected by removing the silica beads. The absorbance of the liquid fraction was measured at wavelengths from 230 to 330 nm using the spectrophotometer. As a control, the complementary strands were added to distilled water to prepare a complementary strand solution with a final complementary strand concentration of 25 µmol/l, and the absorbance of this complementary strand solution was measured.

The collected liquid fraction contained the complementary strands not bound to the immobilized aptamers. Thus, it can be said that the absorbance of the liquid fraction is the absorbance (C) of the unbound complementary strands. On the other hand, it can be said that the absorbance of the complementary strand solution with the final complementary strand concentration of 25 µmol/l corresponds to the absorbance (I) of all the complementary strands before being brought into contact with the immobilized aptamers. Thus, by dividing the absorbance (C) of the collected complementary strands in the liquid fraction by the absorbance (I) of the complementary strands before reacting with the immobilized aptamers, the proportion of the collected complementary strands (C) to the complementary strands (I) added initially (100 × [C/I], unit: %) was determined.

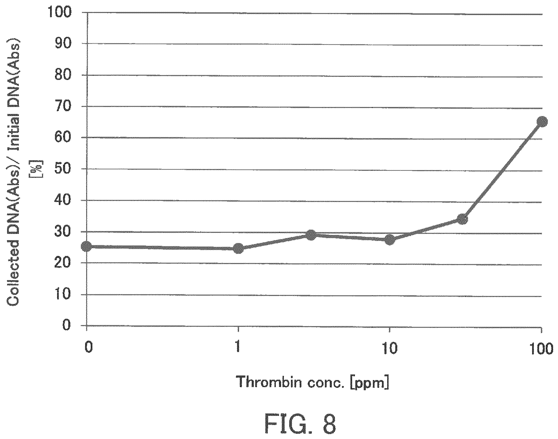

The results thereof are shown in FIG. 8. FIG. 8 is a graph showing the relationship between the concentration of the human α-thrombin and the above-described proportion (%). As can be seen in FIG. 8, the amount of the collected complementary strands increased in keeping with the increase in the concentration of the thrombin as the target protein. This demonstrates that it is possible to detect a target by using aptamers and complementary strands complementary to the aptamers.

### (Example 3)

The target analysis tool in the second A-form is configured so that, for example, as shown in FIG. 4, a target 18 in a specimen held by the specimen holder 17 is caused to bind to the aptamers 241 in the labeled aptamers 24 in the first chamber 11, and among the labeled aptamers 24, those not bound to the target 18 are caused to bind to the immobilized complementary strands 25 in the second chamber 12. Thus, the present example examined whether the amount of a target can be measured by collecting a specimen with a cotton swab of the specimen holder 17 and causing reactions in the above-described order.

### (1) Specimen

Commercially available peanuts (seeds) were ground with a mill. 5 g of the thus-obtained powder was mixed with 20 ml of a SB1T buffer solution, and the resultant mixed solution was shaken for 1 minute using a shaker at 90 to 110 rpm at room temperature (around 25°C, the same applies hereinafter). The SB1T buffer solution had the following composition: 40 mmol/l HEPES (pH 7.4), 125 mmol/l NaCl, 5 mmol/l KCl, 1 mmol/l MgCl₂, and 0.005 (v/v)% Tween® 20.

The mixed solution after being shaken was centrifuged for 30 minutes at 10000 g at room temperature. Then, the supernatant was collected, and the supernatant was filtered through a filter (pore size: 0.8 µm) to prepare a peanut extract. Then, the protein concentration in the peanut extract was quantified using a protein concentration measurement kit (Protein Assay reagent, Bio-Rad).

### (2) Labeled aptamers

Labeled aptamers were prepared by binding biotinylated aptamers to streptavidin-added luciferase (Streptavidin Lucia, Invivogen). As the aptamers, peanut aptamers that bind to peanut allergens were used. The peanut aptamer is represented by the following sequence and has biotin added to the 5' end. The labeled aptamers were prepared by immobilizing 400 pmol of the aptamers per 100 pmol of the streptavidin.
Peanut aptamer (SEQ ID NO: 4)

### (3) Immobilized complementary strands

Immobilized complementary strands were prepared by binding complementary strands to beads. As the beads, magnetic beads having streptavidin added thereto (MyOne SA beads, Invirogen) were used. As the aptamers, complementary strands each represented by the following sequence and having biotin added to the 5' end were used. The immobilized complementary strands were prepared by immobilizing 500 pmol of the complementary strands per mg of the magnetic beads.
Complementary strand (SEQ ID NO: 5)
5'-AAAAAAAAAAAAAAAAAAAATAGAGAGTCAGTCCCAACCA-3'

### (4) Method

The labeled aptamers were added to the buffer solution to prepare a labeled aptamer solution. The immobilized complementary strands were added to distilled water to prepare an immobilized complementary strand solution.

Samples were prepared by diluting the peanut extract with the SB1T buffer solution so as to achieve predetermined peanut protein concentrations (0, 1, 10, 100, 1000, and 7700 ppm). 100 µl of each sample was applied to aluminum foil. Thereafter, the aluminum foil was wiped with a cotton swab. The cotton swab was brought into contact with 400 µl of the SB1T buffer solution. The resultant mixed solution was incubated at room temperature for 1 minute, whereby the target held by the cotton swab was extracted. Thus, an extract was obtained. 25 µl of the labeled aptamer solution was added to 25 µl of the extract so that the amount of the aptamers derived from the labeled aptamers was 1 pmol. The resultant mixed solution was stirred at room temperature for 1 minute, thereby causing the labeled aptamers to bind to the peanut allergen as the target protein. To this mixed solution, 4 µl of the immobilized complementary strand solution (the complementary strands: 40 pmol) was further added so that the resultant mixed solution contained the same amount of the complementary strands (10 µmol/l) as the aptamers derived from the labeled aptamers. The resultant mixed solution was stirred at room temperature for 4 minutes, thereby causing the immobilized complementary strands to bind to the aptamers in the labeled aptamers not bound to the target protein.

Next, the resultant mixed solution was set in a magnetic holder to separate the mixed solution into a fraction of the magnetic beads and a fraction of components other than the magnetic beads, and the latter fraction was collected. Then, 30 µl of a substrate solution (Quanti-Luc (trademark), Invivogen) was added to 30 µl of the supernatant, and the resultant mixed solution was pipetted. Thereafter, the amount of light emission in each sample was measured using a plate reader (Infinite M1000 Pro, TECAN).

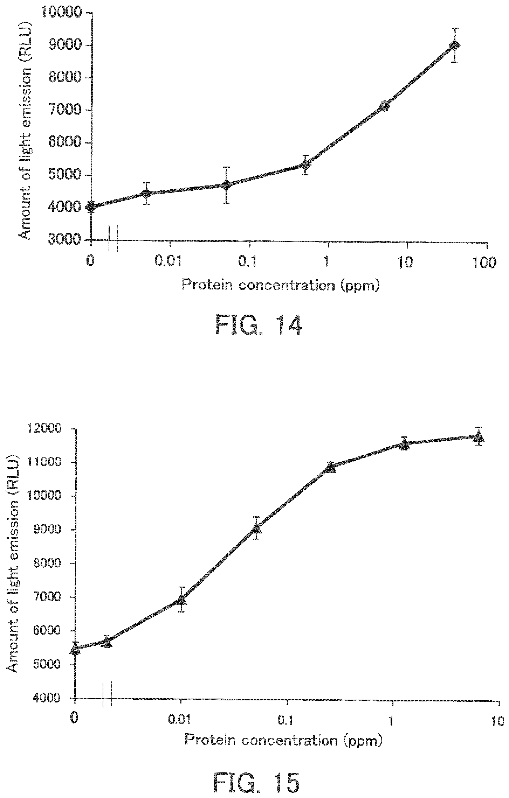

The results thereof are shown in FIG. 14. FIG. 14 is a graph showing the amount of light emission. In FIG. 14, the horizontal axis indicates the peanut protein concentration in the mixed solution obtained by mixing the extract with the labeled aptamers, and the vertical axis indicates the amount of light emission. As can be seen in FIG. 14, the amount of light emission increased in a peanut protein concentration dependent manner. This demonstrates that the amount of a target can be measured by collecting a specimen with a cotton swab of the specimen holder 17 and causing reactions in the above-described order.

### (Example 4)

The target analysis tool in the second A-form is configured so that, for example, as shown in FIG. 4, a target 18 in a specimen held by the specimen holder 17 is caused to bind to the aptamers 241 in the labeled aptamers 24 in the first chamber 11, and among the labeled aptamers 24, those not bound to the target 18 are caused to bind to the immobilized complementary strands 25 in the second chamber 12. Thereafter, only first conjugates of the labeled aptamers 24 and the target 18 are introduced to the third chamber. Thus, the present example examined whether the amount of a target can be measured by causing reactions in the above-described order, then causing the mixture of the labeled aptamers 24, the immobilized complementary strands 25, and the target 18 to pass through a filter of the second partition wall 121, and detecting the first conjugates in the thus-obtained solution.

First, a peanut extract, a labeled aptamer solution, and an immobilized complementary strand solution were prepared in the same manner as in Example 3, except that, instead of the magnetic beads, resin beads having streptavidin added thereto (Pierce (trademark) Streptavidin Plus UltraLink (trademark) Resin, PIERCE) were used.

Samples were prepared by diluting the peanut extract with the SB1T buffer solution so as to achieve predetermined peanut protein concentrations (0, 0.002, 0.01, 0.05, 0.25, 1.25, and 6.25 ppm). 25 µl of the labeled aptamer solution was added to 25 µl of the extract so that the amount of the aptamers derived from the labeled aptamers was 1 pmol. The resultant mixture was stirred at room temperature for 1 minute, thereby causing the labeled aptamers to bind to the peanut allergen as the target protein. To this mixed solution, 10 µl of the immobilized complementary strand solution (the complementary strands: 40 pmol) was further added so that the resultant mixed solution contained the same amount of the complementary strands (4 µmol/l) as the aptamers derived from the labeled aptamers. The resultant mixed solution was stirred at room temperature for 4 minutes, thereby causing the immobilized complementary strands to bind to the aptamers in the labeled aptamers not bound to the target protein. The resultant mixed solution was subjected to centrifugal filtration using a filter plate (pore size: 0.22 µm, MultiScreen®, Millipore Corporation). 30 µl of the substrate solution was added to 30 µl of the thus-obtained filtrate, and the resultant mixed solution was pipetted. Thereafter, the amount of light emission in each sample was measured using the plate reader.

The results thereof are shown in FIG. 15. FIG. 15 is a graph showing the amount of light emission. In FIG. 15, the horizontal axis indicates the peanut protein concentration, and the vertical axis indicates the amount of light emission. As can be seen in FIG. 15, the amount of light emission increased in a peanut protein concentration dependent manner. This demonstrates that the amount of a target can be measured by causing reactions in the above-described order, then causing the mixture of the labeled aptamers 24, the immobilized complementary strands 25, and the target 18 to pass through a filter of the second partition wall 121, and detecting the first conjugates in the thus-obtained solution.

### (Example 5)

The target analysis tool in the fourth A-form according to the invention is configured so that, for example, as shown in FIG. 13, a target 18 in a specimen held by the specimen holder 17 is mixed with the immobilized complementary strands 25 in the first chamber 11, and in the second chamber 12, the target 18 in the contents in the first chamber 11 is caused to bind to the labeled aptamers 24, and among the labeled aptamers 24, those not bound to the target 18 are caused to bind to the immobilized complementary strands 25. Thus, the present example examined whether the amount of a target can be measured by causing reactions in the above-described order.

First, a peanut extract, a labeled aptamer solution, and an immobilized complementary strand solution were prepared in the same manner as in Example 3.

Samples were prepared by diluting the peanut extract with the SB1T buffer solution so as to achieve predetermined peanut protein concentrations (0, 0.002, 0.01, 0.05, 0.25, 1.25, and 6.25). Thus, samples were prepared. 25 µl of the immobilized complementary strand solution was added to 25 µl of each sample so that the amount of the complementary strands derived from the immobilized complementary strands was 1 pmol, and the resultant mixed solution was stirred at room temperature for 1 minute. To this mixed solution, 4 µl of the labeled aptamer solution (the aptamers: 40 pmol) was further added so that the resultant mixed solution contained the same amount of the aptamers (10 µmol/l) as the complementary strands derived from the immobilized complementary strands. The resultant mixed solution was stirred at room temperature for 4 minutes, thereby causing the labeled aptamers to bind to the peanut allergen as the target protein and also causing the immobilized complementary strands to bind to the aptamers in the labeled aptamers not bound to the target protein.

Next, the resultant mixed solution was set in a magnetic holder to separate the mixed solution into a fraction of the magnetic beads and a fraction of components other than the magnetic beads, and the latter fraction was collected. Then, 30 µl of the substrate solution was added to 30 µl of the supernatant, and the resultant mixed solution was pipetted. Thereafter, the amount of light emission in each sample was measured using the plate reader.

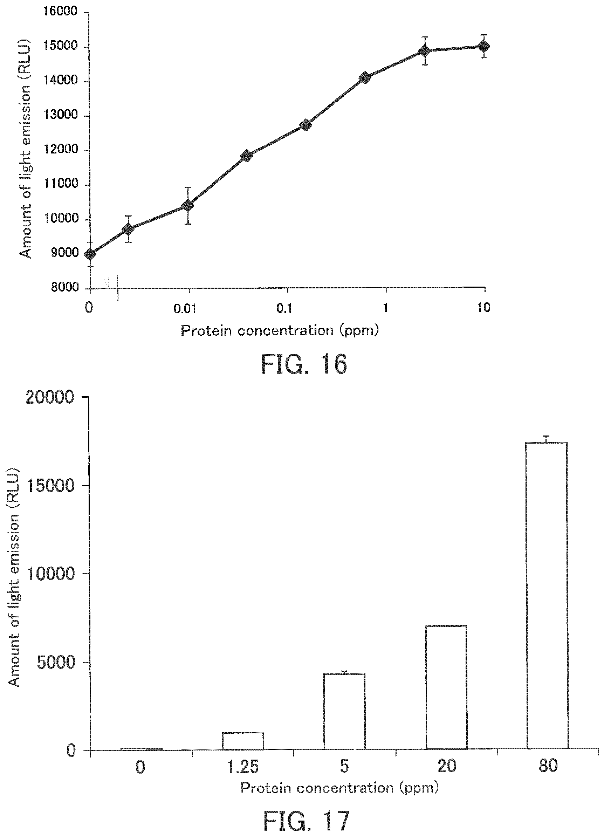

The results thereof are shown in FIG. 16. FIG. 16 is a graph showing the amount of light emission. In FIG. 16, the horizontal axis indicates the peanut protein concentration, and the vertical axis indicates the amount of light emission. As can be seen in FIG. 16, the amount of light emission increased in a peanut protein concentration dependent manner. This demonstrates that the amount of a target can be measured by causing reactions in the above-described order.

### (Example 6)

The target analysis tool in the second A-form is configured so that, for example, as shown in FIG. 4, a target 18 in a specimen held by the specimen holder 17 is caused to bind to the aptamers 241 in the labeled aptamers 24 in the first chamber 11, and among the labeled aptamers 24, those not bound to the target 18 are caused to bind to the immobilized complementary strands 25 in the second chamber 12. Thereafter, only first conjugates of the labeled aptamers 24 and the target 18 are introduced to the third chamber. Thus, the present example examined whether the amount of a target can be measured by causing reactions in the above-described order, then causing the mixture of the labeled aptamers 24, the immobilized complementary strands 25, and the target 18 to pass through a filter of the second partition wall 121, and detecting the first conjugates in the thus-obtained solution.

A peanut extract 2 was prepared and the protein concentration therein was quantified in the same manner as in Example 3, except that the powder of the peanut was mixed with the SB1T buffer solution and then the resultant mixed solution was shaken overnight (for about 16 hours). Also, a labeled aptamer solution and an immobilized complementary strand solution were prepared in the same manner as in Example 3.

Peanut diluted solutions were prepared by diluting the peanut extract 2 with the SB IT buffer solution so as to achieve predetermined protein concentrations (0, 1.25, 5, 20, and 80 ppm) in a reaction solution obtained after being mixed with the labeled aptamer solution and the immobilized complementary strand solution. Next, a diluted immobilized complementary strand solution was prepared by adding 40 µl of the immobilized complementary strand solution and 160 µl of the SB IT buffer solution to a tube. To another tube, 250 µl of the labeled aptamer solution was added, and 250 µl of each peanut diluted solution was further added. After the addition, the tube was agitated. Further, 200 µl of the diluted immobilized complementary strand solution was added to the tube. Thereafter, the tube was then agitated, and then incubated at room temperature for 1 minute.

The reaction solution after the incubation was filtered using a 5 ml syringe and a filter (pore size: 0.45 µm), and the filtrate was collected in a measurement container to which 800 µl of the substrate solution had been added beforehand. After the collection, the measurement container was agitated, and incubated for 30 seconds to 1 minute. Then, using a luminometer (Clean-Trace (trademark), 3M Co.), the amount of light emission in each measurement sample in the measurement container was measured.

The results thereof are shown in FIG. 17. FIG. 17 is a graph showing the amount of light emission. In FIG. 17, the horizontal axis indicates the protein concentration, and the vertical axis indicates the amount of light emission. As can be seen in FIG. 17, the amount of light emission increased in a peanut protein concentration dependent manner. This demonstrates that the amount of a target can be measured by causing reactions in the above-described order, then causing the mixture of the labeled aptamers 24, the immobilized complementary strands 25, and the target 18 to pass through a filter of the second partition wall 121, and detecting the first conjugates in the thus-obtained solution.

This application claims priority from Japanese Patent Application No. 2015-010514 filed on January 22, 2015 and Japanese Patent Application No. 2015-145280 filed on July 22, 2015.

### Industrial Applicability

According to the present invention, a target in a specimen can be analyzed easily.

### Explanation of Reference Numerals

- 1, 2, 3, 4, 10:: target analysis tool
- 11, 92:: first chamber
- 12, 93:: second chamber
- 13, 94:: third chamber
- 14:: immobilized aptamer
- 15:: labeled complementary strand
- 24:: labeled aptamer
- 25:: immobilized complementary strand
- 141, 241:: aptamer
- 142,252:: bead
- 151, 251:: complementary strand
- 152, 242:: DNAzyme
- 16:: silica bead
- 17:: specimen holder
- 171:: grip section
- 172:: holding section
- 18:: target
- 19:: contaminant
- 91:: pretreatment chamber
- 95:: substrate
- 96:: extractant
- 111, 921:: first partition wall
- 121, 931:: second partition wall
- 901:: cover
- 911:: partition wall

### [Sequence Listing]

2016.01.19_TF14098WO_ST25.txt

## Claims

1. A target analysis tool comprising:
a first chamber;
a second chamber; and
a third chamber,
wherein the first chamber, the second chamber, and the third chamber are disposed continuously in this order,
the first chamber contains, as a first reagent, an immobilized second binding substance obtained by immobilizing, on a carrier, a second binding substance that binds to a first binding substance that binds to a target,
the second chamber contains, as a second reagent, a labeled first binding substance obtained by binding a labeling substance to the first binding substance,
the third chamber is a detection section in which the labeled first binding substance is detected,
a first partition wall is provided between the first chamber and the second chamber,
a second partition wall is provided between the second chamber and the third chamber,
the first chamber is configured so that a specimen holder having a specimen can be inserted from an outside of the first chamber to an inside of the first chamber,
the first partition wall is a partition wall that is broken upon contact with a tip of the specimen holder inserted into the first chamber, and
the second partition wall is a porous partition wall through which the immobilized second binding substance cannot pass and the labeled first binding substance not bound to the immobilized second binding substance can pass, wherein
the first binding substance in the labeled first binding substance is an aptamer, and the second binding substance in the immobilized second binding substance is a nucleic acid molecule complementary to the aptamer.

2. The target analysis tool according to claim 1, wherein
the labeling substance in the labeled first binding substance is at least one substance selected from the group consisting of enzymes, nucleic acids, fluorescent substances, dye substances, luminescent substances, radioactive substances, and electron donors.

3. The target analysis tool according to any one of claims 1 to 2, wherein
the carrier in the immobilized second binding substance is a bead.

4. The target analysis tool according to any one of claims 1 to 3, wherein
the specimen holder comprises a rod-shaped grip section and a holding section for holding a specimen, and
the holding section is provided at a tip of the grip section.

5. The target analysis tool according to any one of claims 1 to 4, wherein
the target analysis tool comprises an outer cylinder and an inner cylinder,
the inner cylinder can be housed inside the outer cylinder,
the inner cylinder has the first chamber and the second chamber, and
when the inner cylinder is housed inside the outer cylinder, there is a space between a bottom of the outer cylinder and a bottom of the inner cylinder.

6. The target analysis tool according to any one of claims 1 to 5, further comprising a pretreatment chamber disposed continuously with the first chamber on a side opposite to the second chamber, wherein
the pretreatment chamber is configured so that the specimen holder can be inserted from an outside of the pretreatment chamber to an inside of the pretreatment chamber,
the pretreatment chamber contains an extractant for extracting a component from the specimen,
a partition wall is provided between the pretreatment chamber and the first chamber, and
the partition wall is a partition wall that is broken upon contact with the tip of the specimen holder inserted into the pretreatment chamber.

7. The target analysis tool according to claim 6, wherein
the pretreatment chamber comprises a cover for an opening of the pretreatment chamber on a side opposite to the partition wall between the pretreatment chamber and the first chamber, and
the cover is configured so that the specimen holder can be inserted from an outside of the cover to an inside of the pretreatment chamber.

8. A target analysis method using the target analysis tool according to any one of claims 1 to 7, the target analysis method comprising:
introducing a specimen and the first reagent to the second chamber by inserting a specimen holder holding the specimen into the first chamber of the target analysis tool and then bringing the specimen holder into contact with the first partition wall between the first chamber and the second chamber;
binding a target in the specimen to the labeled first binding substance as the second reagent to form a first conjugate and also binding an unbound labeled first binding substance not bound to the target to the immobilized second binding substance as the first reagent by causing the specimen and the first reagent to be in contact with the second reagent in the second chamber;
introducing the first conjugate to the third chamber through the second partition wall between the second chamber and the third chamber; and
detecting the labeled first binding substance in the first conjugate in the third chamber.

9. The target analysis method according to claim 8, further comprising the steps of:
mixing the specimen and the first reagent by inserting the specimen holder into the first chamber; and
introducing a mixture of the specimen and the first reagent in the first chamber to the second chamber by bringing the specimen holder in the first chamber into contact with the first partition wall between the first chamber and the second chamber to break the partition wall.

## Patentansprüche

1. Targetanalysewerkzeug, umfassend folgendes:
eine erste Kammer;
eine zweite Kammer; und
eine dritte Kammer,
wobei die erste Kammer, die zweite Kammer und die dritte Kammer durchgehend in dieser Reihenfolge angeordnet sind,
wobei die erste Kammer als ein erstes Reagenz eine immobilisierte zweite Bindungssubstanz enthält, erhalten durch Immobilisieren, auf einem Träger, einer zweiten Bindungssubstanz, die an eine erste Bindungssubstanz bindet, die an ein Target bindet,
wobei die zweite Kammer als ein zweites Reagenz eine markierte erste Bindungssubstanz enthält, erhalten durch Binden einer Markierungssubstanz an die erste Bindungssubstanz,
wobei die dritte Kammer ein Detektionsabschnitt ist, in dem die markierte erste Bindungssubstanz detektiert wird,
wobei eine erste Trennwand zwischen der ersten Kammer und der zweiten Kammer vorgesehen ist,
wobei eine zweite Trennwand zwischen der zweiten Kammer und der dritten Kammer vorgesehen ist,
wobei die erste Kammer derart konfiguriert ist, dass ein Probenhalter mit einer Probe von einer Außenseite der ersten Kammer zu einer Innenseite der ersten Kammer eingesetzt werden kann,
wobei die erste Trennwand eine Trennwand ist, die bei Kontakt mit einer Spitze des Probenhalters, der in die erste Kammer eingesetzt wird, aufgebrochen wird, und
wobei die zweite Trennwand eine poröse Trennwand ist, durch die die immobilisierte zweite Bindungssubstanz nicht hindurch passieren kann, und die markierte erste Bindungssubstanz, die nicht an die immobilisierte zweite Bindungssubstanz gebunden ist, passieren kann, wobei die erste Bindungssubstanz in der markierten ersten Bindungssubstanz ein Aptamer ist, und wobei die zweite Bindungssubstanz in der immobilisierten zweiten Bindungssubstanz ein zu dem Aptamer komplementäres Nukleinsäuremolekül ist.

2. Targetanalysewerkzeug nach Anspruch 1, wobei
die Markierungssubstanz in der markierten ersten Bindungssubstanz wenigstens eine Substanz ist, ausgewählt aus der Gruppe bestehend aus Enzymen, Nukleinsäuren, fluoreszierenden Substanzen, Färbesubstanzen, lumineszierenden Substanzen, radioaktiven Substanzen und Elektronendonatoren.

3. Targetanalysewerkzeug nach einem der Ansprüche 1 bis 2, wobei der Träger in der immobilisierten zweiten Bindungssubstanz eine Kugel ist.

4. Targetanalysewerkzeug nach einem der Ansprüche 1 bis 3, wobei der Probenhalter einen stangenförmigen Griffabschnitt und einen Halteabschnitt zum Halten einer Probe umfasst, und
wobei der Halteabschnitt an einer Spitze des Griffabschnitts vorgesehen ist.

5. Targetanalysewerkzeug nach einem der Ansprüche 1 bis 4, wobei
das Targetanalysewerkzeug einen äußeren Zylinder und einen inneren Zylinder umfasst,
der innere Zylinder innerhalb des äußeren Zylinders untergebracht werden kann,
der innere Zylinder die erste Kammer und die zweite Kammer hat, und
wenn der innere Zylinder innerhalb des äußeren Zylinders untergebracht ist, es einen Raum gibt zwischen einem Boden des äußeren Zylinders und einem Boden des inneren Zylinders.

6. Targetanalysewerkzeug nach einem der Ansprüche 1 bis 5, ferner umfassend eine Vorbehandlungskammer, die durchgehend mit der ersten Kammer an einer Seite entgegengesetzt zu der zweiten Kammer angeordnet ist, wobei
die Vorbehandlungskammer derart konfiguriert ist, dass der Probenhalter von einer Außenseite der Vorbehandlungskammer zu einer Innenseite der Vorbehandlungskammer eingesetzt werden kann,
die Vorbehandlungskammer ein Extraktionsmittel zum Extrahieren einer Komponente von der Probe enthält,
eine Trennwand zwischen der Vorbehandlungskammer und der ersten Kammer vorgesehen ist, und
die Trennwand eine Trennwand ist, die bei Kontakt mit der Spitze des Probenhalters, der in die Vorbehandlungskammer eingesetzt wird, aufgebrochen wird.

7. Targetanalysewerkzeug nach Anspruch 6, wobei
die Vorbehandlungskammer eine Abdeckung für eine Öffnung der Vorbehandlungskammer an einer Seite entgegengesetzt zu der Trennwand zwischen der Vorbehandlungskammer und der ersten Kammer umfasst, und
die Abdeckung derart konfiguriert ist, dass der Probenhalter von einer Außenseite der Abdeckung zu einer Innenseite der Vorbehandlungskammer eingesetzt werden kann.

8. Targetanalyseverfahren unter Verwendung des Targetanalysewerkzeugs nach einem der Ansprüche 1 bis 7, wobei das Targetanalyseverfahren folgendes umfasst:
Einführen einer Probe und des ersten Reagenz zu der zweiten Kammer durch Einsetzen eines Probenhalters, der die Probe hält, in die erste Kammer des Targetanalysewerkzeugs und dann in Kontakt bringen des Probenhalters mit der ersten Trennwand zwischen der ersten Kammer und der zweiten Kammer;
Binden eines Targets in der Probe an die markierte erste Bindungssubstanz als das zweite Reagenz zum Bilden eines ersten Konjugats, und ferner Binden einer ungebundenen markierten ersten Bindungssubstanz, die nicht an das Target gebunden ist, an die immobilisierte zweite Bindungssubstanz als das erste Reagenz durch Veranlassen, dass die Probe und das erste Reagenz in Kontakt mit dem zweiten Reagenz in der zweiten Kammer sind;
Einführen des ersten Konjugats zu der dritten Kammer durch die zweite Trennwand zwischen der zweiten Kammer und der dritten Kammer hindurch; und
Detektieren der markierten ersten Bindungssubstanz in dem ersten Konjugat in der dritten Kammer,

9. Targetanalyseverfahren nach Anspruch 8, ferner umfassend die folgenden Schritte:
Mischen der Probe und des ersten Reagenz durch Einsetzen des Probenhalters in die erste Kammer; und
Einführen einer Mischung der Probe und des ersten Reagenz in der ersten Kammer zu der zweiten Kammer durch in Kontakt bringen des Probenhalters in der ersten Kammer mit der ersten Trennwand zwischen der ersten Kammer und der zweiten Kammer, um die Trennwand zu durchbrechen.

## Revendications

1. Outil d'analyse de cible comprenant :
une première chambre ;
une deuxième chambre ; et
une troisième chambre,
dans lequel la première chambre, la deuxième chambre, et la troisième chambre sont disposées en continu dans cet ordre,
la première chambre contient, en tant que premier réactif, une deuxième substance liante immobilisée obtenue en immobilisant, sur un support, une deuxième substance liante qui se lie à une première substance liante qui se lie à une cible,
la deuxième chambre contient, en tant que deuxième réactif, une première substance liante marquée obtenue en liant une substance de marquage à la première substance liante,
la troisième chambre est une section de détection dans laquelle la première substance liante marquée est détectée,
une première cloison de séparation est prévue entre la première chambre et la deuxième chambre,
une deuxième cloison de séparation est prévue entre la deuxième chambre et la troisième chambre,
la première chambre est configurée de sorte qu'un porte-échantillon ayant un échantillon puisse être inséré depuis un extérieur de la première chambre jusqu'à un intérieur de la première chambre,
la première cloison de séparation est une cloison de séparation qui est rompue lors d'un contact avec un bout du porte-échantillon inséré dans la première chambre, et
la deuxième cloison de séparation est une cloison de séparation poreuse à travers laquelle la deuxième substance liante immobilisée ne peut pas passer et la première substance liante marquée non liée à la deuxième substance liante immobilisée peut passer, dans lequel
la première substance liante dans la première substance liante marquée est un aptamère, et la deuxième substance liante dans la deuxième substance liante immobilisée est une molécule d'acide nucléique complémentaire de l'aptamère.

2. Outil d'analyse de cible selon la revendication 1, dans lequel
la substance de marquage dans la première substance liante marquée est au moins une substance choisie dans le groupe consistant en des enzymes, des acides nucléiques, des substances fluorescentes, des substances colorantes, des substances luminescentes, des substances radioactives, et des donneurs d'électron.

3. Outil d'analyse de cible selon l'une quelconque des revendications 1 et 2, dans lequel
le support dans la deuxième substance liante immobilisée est une bille.

4. Outil d'analyse de cible selon l'une quelconque des revendications 1 à 3, dans lequel
le porte-échantillon comprend une section de préhension en forme de tige et une section d'accueil pour accueillir un échantillon, et
la section d'accueil est prévue au niveau d'un bout de la section de préhension.

5. Outil d'analyse de cible selon l'une quelconque des revendications 1 à 4, dans lequel
l'outil d'analyse de cible comprend un cylindre externe et un cylindre interne,
le cylindre interne peut être logé à l'intérieur du cylindre externe,
le cylindre interne comporte la première chambre et la deuxième chambre, et
lorsque le cylindre interne est logé à l'intérieur du cylindre externe, il y a un espace entre un fond du cylindre externe et un fond du cylindre interne.

6. Outil d'analyse de cible selon l'une quelconque des revendications 1 à 5, comprenant en outre une chambre de prétraitement disposée en continu avec la première chambre sur un côté opposé à la deuxième chambre, dans lequel
la chambre de prétraitement est configurée de sorte que le porte-échantillon puisse être inséré depuis un extérieur de la chambre de prétraitement jusqu'à un intérieur de la chambre de prétraitement,
la chambre de prétraitement contient un agent d'extraction pour extraire un composant de l'échantillon,
une cloison de séparation est prévue entre la chambre de prétraitement et la première chambre, et
la cloison de séparation est une cloison de séparation qui est rompue lors d'un contact avec le bout du porte-échantillon inséré dans la chambre de prétraitement.

7. Outil d'analyse de cible selon la revendication 6, dans lequel
la chambre de prétraitement comprend un couvercle pour une ouverture de la chambre de prétraitement sur un côté opposé à la cloison de séparation entre la chambre de prétraitement et la première chambre, et
le couvercle est configuré de sorte que le porte-échantillon puisse être inséré depuis un extérieur du couvercle jusqu'à un intérieur de la chambre de prétraitement.

8. Procédé d'analyse de cible à l'aide de l'outil d'analyse de cible selon l'une quelconque des revendications 1 à 7, le procédé d'analyse de cible comprenant :
l'introduction d'un échantillon et du premier réactif dans la deuxième chambre en insérant un porte-échantillon accueillant l'échantillon dans la première chambre de l'outil d'analyse de cible puis en amenant le porte-échantillon en contact avec la première cloison de séparation entre la première chambre et la deuxième chambre ;
la liaison d'une cible dans l'échantillon à la première substance liante marquée en tant que deuxième réactif pour former un premier conjugué et la liaison également d'une première substance liante marquée non liée, non liée à la cible à la deuxième substance liante immobilisée en tant que premier réactif en amenant l'échantillon et le premier réactif en contact avec le deuxième réactif dans la deuxième chambre ;
l'introduction du premier conjugué jusqu'à la troisième chambre à travers la deuxième cloison de séparation entre la deuxième chambre et la troisième chambre ; et
la détection de la première substance liante marquée dans le premier conjugué dans la troisième chambre.

9. Procédé d'analyse de cible selon la revendication 8, comprenant en outre les étapes de :
mélange de l'échantillon et du premier réactif en insérant le porte-échantillon dans la première chambre ; et
introduction d'un mélange de l'échantillon et du premier réactif dans la première chambre jusqu'à la deuxième chambre en amenant le porte-échantillon dans la première chambre en contact avec la première cloison de séparation entre la première chambre et la deuxième chambre pour rompre la cloison de séparation.


### SEQUENCE LISTING

<110> NEC Solution Innovators, Ltd.
<120> Apparatus of analyzing target and method of analyzing target
<130> 1010100036
<150> JP 2015-010514
   <151> 2015-01-22
<150> JP 2015-145280
   <151> 2015-07-22
<160> 5
<170> PatentIn version 3.5
<210> 1
   <211> 20
   <212> DNA
   <213> Artificial Sequence
<220>
   <223> polynucleotide
<400> 1
   aaaaaccaac cacaccaacc 20
<210> 2
   <211> 20
   <212> DNA
   <213> Artificial Sequence
<220>
   <223> aptamer
<400> 2
   ggttggtgtg gttggttttt 20
<210> 3
   <211> 20
   <212> DNA
   <213> Artificial Sequence
<220>
   <223> complementary polynucleotide
<400> 3
   aaaaaccaac cacaccaacc 20
<210> 4
   <211> 79
   <212> DNA
   <213> Artificial Sequence
<220>
   <223> aptamer
<400> 4
   ggatattgcc tcgccacagt taagtcaggt ggttggttat ggttgggact gactctctac 60
   agggaacgct cggattatc 79
<210> 5
   <211> 40
   <212> DNA
   <213> Artificial Sequence
<220>
   <223> complementary polynucleotide
<400> 5
   aaaaaaaaaa aaaaaaaaaa tagagagtca gtcccaacca 40